# EUROPEAN PATENT SPECIFICATION

(11) **EP 2 619 318 B1**
(45) Date of publication and mention of the grant of the patent: **24.12.2014**
(21) Application number: 11757670.2
(22) Date of filing: 20.09.2011
(51) Int. Cl.: C12Q 1/68

(54) **MICROWAVE-DRIVEN RNA POLYMERIZATION BY RNA POLYMERASES OF CALICIVIRUSES**
DURCH MIKROWELLEN INDUZIERTE RNA POLYMERISATION MITTELS RNA POLYMERASEN VON CALICIVIREN
RNA-POLYMÉRISATION PAR ARN-POLYMÉRASES DES CALICIVIRUS INDUCÉE PAR MICRO-ONDES

(30) Priority: 21.09.2010 EP 10178114
(43) Date of publication of application: 31.07.2013
(73) Proprietor: RiboxX GmbH, 01445 Radebeul (DE)
(72) Inventor: ROHAYEM, Jacques, 01277 Dresden (DE)
(74) Representative: Habermann, Hruschka & Schnabel
(86) International application number: PCT/EP2011/066362
(87) International publication number: WO 2012/038450

(56) References cited:
- EP-A1- 1 840 226
- WO-A1-93/21344
- WO-A1-2009/150156
- WO-A2-2007/012329
- FERMER C ET AL: "Microwave-assisted high-speed PCR", EUROPEAN JOURNAL OF PHARMACEUTICAL SCIENCES, ELSEVIER, AMSTERDAM, NL, vol. 18, no. 2, 1 February 2003 (2003-02-01), pages 129-132, XP002436821, ISSN: 0928-0987, DOI: 10.1016/S0928-0987(02)00252-X cited in the application

### SEQUENCE LISTING

<110> RiboxX GmbH
<120> Microwave-driven RNA polymerization by RNA polymerases of caliciviruses
<130> R 0076 WO
<150> EP 10 178 114.4
   <151> 2010-09-21
<160> 17
<170> PatentIn version 3.3
<210> 1
   <211> 5
   <212> PRT
   <213> Artificial
<220>
   <223> A-motif
<220>
   <221> misc_feature
   <222> (1)..(2)
   <223> Xaa = any amino acid
<400> 1
   Xaa Xaa Asp Tyr Ser
   1               5
<210> 2
   <211> 5
   <212> PRT
   <213> Artificial
<220>
   <223> B-motif
<220>
   <221> misc_feature
   <222> (2)..(2)
   <223> Xaa = any amino acid
<400> 2
   Gly Xaa Pro Ser Gly
   1               5
<210> 3
   <211> 4
   <212> PRT
   <213> Artificial
<220>
   <223> C-motif
<400> 3
   Tyr Gly Asp Asp
   1
<210> 4
   <211> 5
   <212> PRT
   <213> Artificial
<220>
   <223> D-motif
<220>
   <221> misc_feature
   <222> (1)..(2)
   <223> Xaa = any amino acid
<400> 4
   Xaa Xaa Tyr Gly Leu
   1 5
<210> 5
   <211> 10
   <212> PRT
   <213> Artificial
<220>
   <223> E-Motif
<220>
   <221> misc_feature
   <222> (1)..(4)
   <223> Xaa = any amino acid
<220>
   <221> misc_feature
   <222> (7)..(7)
   <223> Xaa = any amino acid
<220>
   <221> misc_feature
   <222> (9)..(10)
   <223> Xaa = any amino acid
<400> 5
   Xaa Xaa Xaa Xaa Phe Leu Xaa Arg Xaa Xaa
   1 5 10
<210> 6
   <211> 520
   <212> PRT
   <213> Norovirus
<400> 6
   Met Gly Gly Asp Ser Lys Gly Thr Tyr Cys Gly Ala Pro Ile Leu Gly
   1               5                   10                  15
   Pro Gly Ser Ala Pro Lys Leu Ser Thr Lys Thr Lys Phe Trp Arg Ser
   20 25 30
   Ser Thr Thr Pro Leu Pro Pro Gly Thr Tyr Glu Pro Ala Tyr Leu Gly
   35 40 45
   Gly Lys Asp Pro Arg Val Lys Gly Gly Pro Ser Leu Gln Gln Val Met
   50 55 60
   Arg Asp Gln Leu Lys Pro Phe Thr Glu Pro Arg Gly Lys Pro Pro Lys
   65 70 75 80
   Pro Ser Val Leu Glu Ala Ala Lys Lys Thr Ile Ile Asn Val Leu Glu
   85 90 95
   Gln Thr Ile Asp Pro Pro Glu Lys Trp Ser Phe Thr Gln Ala Cys Ala
   100 105 110
   Ser Leu Asp Lys Thr Thr Ser Ser Gly His Pro His His Met Arg Lys
   115 120 125
   Asn Asp Cys Trp Asn Gly Glu Ser Phe Thr Gly Lys Leu Ala Asp Gln
   130 135 140
   Ala Ser Lys Ala Asn Leu Met Phe Glu Gly Gly Lys Asn Met Thr Pro
   145 150 155 160
   Val Tyr Thr Gly Ala Leu Lys Asp Glu Leu Val Lys Thr Asp Lys Ile
   165 170 175
   Tyr Gly Lys Ile Lys Lys Arg Leu Leu Trp Gly Ser Asp Leu Ala Thr
   180 185 190
   Met Ile Arg Cys Ala Arg Ala Phe Gly Gly Leu Met Asp Glu Leu Lys
   195 200 205
   Ala His Cys Val Thr Leu Pro Ile Arg Val Gly Met Asn Met Asn Glu
   210 215 220
   Asp Gly Pro Ile Ile Phe Glu Arg His Ser Arg Tyr Lys Tyr His Tyr
   225 230 235 240
   Asp Ala Asp Tyr Ser Arg Trp Asp Ser Thr Gln Gln Arg Ala Val Leu
   245 250 255
   Ala Ala Ala Leu Glu Ile Met Val Lys Phe Ser Ser Glu Pro His Leu
   260 265 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465                 470                 475                 480
   Pro Met Phe Arg Trp Met Arg Phe Ser Asp Leu Ser Thr Trp Glu Gly
                   485                 490                 495
   Asp Arg Asn Leu Ala Pro Ser Phe Val Asn Glu Asp Gly Val Glu Val
               500                 505                 510
   Asp Lys Leu Ala Ala Ala Leu Glu
           515                 520
<210> 7
   <211> 517
   <212> PRT
   <213> Sapovirus
<400> 7
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
       210                 215                 220
   Ser Val Gln Met Gln Val Met Asn Asp Ser Leu Lys Gly Gly Val Leu
   225                 230                 235                 240
   Tyr Cys Leu Asp Tyr Ser Lys Trp Asp Ser Thr Gln Asn Pro Ala Val
                   245                 250                 255
   Thr Ala Ala Ser Leu Ala Ile Leu Glu Arg Phe Ala Glu Pro His Pro
               260                 265                 270
   Ile Val Ser Cys Ala Ile Glu Ala Leu Ser Ser Pro Ala Glu Gly Tyr
           275                 280                 285
   Val Asn Asp Ile Lys Phe Val Thr Arg Gly Gly Leu Pro Ser Gly Met
       290                 295                 300
   Pro Phe Thr Ser Val Val Asn Ser Ile Asn His Met Ile Tyr Val Ala
   305                 310                 315                 320
   Ala Ala Ile Leu Gln Ala Tyr Glu Ser His Asn Val Pro Tyr Thr Gly
                   325                 330                 335
   Asn Val Phe Gln Val Glu Thr Val His Thr Tyr Gly Asp Asp Cys Met
               340                 345                 350
   Tyr Ser Val Cys Pro Ala Thr Ala Ser Ile Phe His Ala Val Leu Ala
           355                 360                 365
   Asn Leu Thr Ser Tyr Gly Leu Lys Pro Thr Ala Ala Asp Lys Ser Asp
       370                 375                 380
   Ala Ile Lys Pro Thr Asn Thr Pro Val Phe Leu Lys Arg Thr Phe Thr
   385                 390                 395                 400
   Gln Thr Pro His Gly Val Arg Ala Leu Leu Asp Ile Thr Ser Ile Thr
                   405                 410                 415
   Arg Gln Phe Tyr Trp Leu Lys Ala Asn Arg Thr Ser Asp Pro Ser Ser
               420                 425                 430
   Pro Pro Ala Phe Asp Arg Gln Ala Arg Ser Ala Gln Leu Glu Asn Ala
           435                 440                 445
   Leu Ala Tyr Ala Ser Gln His Gly Pro Val Val Phe Asp Thr Val Arg
       450                 455                 460
   Gln Ile Ala Ile Lys Thr Ala Gln Gly Glu Gly Leu Val Leu Val Asn
   465                 470                 475                 480
   Thr Asn Tyr Asp Gln Ala Leu Ala Thr Tyr Asn Ala Trp Phe Ile Gly
                   485                 490                 495
   Gly Thr Val Pro Asp Pro Val Gly His Thr Glu Gly Thr His Lys Ile
   500 505 510
   Val Phe Glu Met Glu
   515
<210> 8
   <211> 533
   <212> PRT
   <213> Vesivirus
<400> 8
   Met Lys Val Thr Thr Gln Lys Tyr Asp Val Thr Lys Pro Asp Ile Ser
   1               5                   10                  15
   Tyr Lys Gly Leu Ile Cys Lys Gln Leu Asp Glu Ile Arg Val Ile Pro
               20                  25                  30
   Lys Gly Thr Arg Leu His Val Ser Pro Ala His Thr Asp Asp Tyr Asp
           35                  40                  45
   Glu Cys Ser His Gln Pro Ala Ser Leu Gly Ser Gly Asp Pro Arg Cys
       50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145                 150                 155                 160
   Ala Thr Gln Gly Ile Gly Leu Pro His Glu Tyr Thr Ile Gly Leu Lys
                   165                 170                 175
   Asp Glu Leu Arg Pro Val Glu Lys Val Gln Glu Gly Lys Arg Arg Met
               180                 185                 190
   Ile Trp Gly Cys Asp Val Gly Val Ala Thr Val Cys Ala Ala Ala Phe
           195                 200                 205
   Lys Gly Val Ser Asp Ala Ile Thr Ala Asn His Gln Tyr Gly Pro Val
   210 215 220
   Gln Val Gly Ile Asn Met Asp Gly Pro Ser Val Glu Ala Leu Tyr Gln
   225 230 235 240
   Arg Ile Arg Ser Ala Ala Lys Val Phe Ala Val Asp Tyr Ser Lys Trp
   245 250 255
   Asp Ser Thr Gln Ser Pro Arg Val Ser Ala Ala Ser Ile Asp Ile Leu
   260 265 270
   Arg Tyr Phe Ser Asp Arg Ser Pro Ile Val Asp Ser Ala Ala Asn Thr
   275 280 285
   Leu Lys Ser Pro Pro Ile Ala Ile Phe Asn Gly Val Ala Val Lys Val
   290 295 300
   Thr Ser Gly Leu Pro Ser Gly Met Pro Leu Thr Ser Val Ile Asn Ser
   305 310 315 320
   Leu Asn His Cys Leu Tyr Val Gly Cys Ala Ile Leu Gln Ser Leu Glu
   325 330 335
   Ser Arg Asn Ile Pro Val Thr Trp Asn Leu Phe Ser Thr Phe Asp Met
   340 345 350
   Met Thr Tyr Gly Asp Asp Gly Val Tyr Met Phe Pro Met Met Phe Ala
   355 360 365
   Ser Val Ser Asp Gln Ile Phe Ala Asn Leu Thr Ala Tyr Gly Leu Lys
   370 375 380
   Pro Thr Arg Val Asp Lys Ser Val Gly Ala Ile Glu Pro Ile Asp Pro
   385 390 395 400
   Glu Ser Val Val Phe Leu Lys Arg Thr Ile Thr Arg Thr Pro His Gly
   405 410 415
   Ile Arg Gly Leu Leu Asp Arg Gly Ser Ile Ile Arg Gln Phe Tyr Tyr
   420 425 430
   Ile Lys Gly Glu Asn Ser Asp Asp Trp Lys Thr Pro Pro Lys Thr Ile
   435 440 445
   Asp Pro Thr Ser Arg Gly Gln Gln Leu Trp Asn Ala Cys Leu Tyr Ala
   450 455 460
   Ser Gln His Gly Pro Glu Phe Tyr Asn Lys Val Tyr Arg Leu Ala Glu
   465 470 475 480
   Lys Ala Val Glu Tyr Glu Glu Leu His Phe Glu Pro Pro Ser Tyr His
   485 490 495
   Ser Ala Leu Glu His Tyr Asn Asn Gln Phe Asn Gly Val Asp Thr Arg
   500 505 510
   Ser Asp Gln Ile Asp Ala Ser Val Met Thr Asp Leu His Cys Asp Val
   515 520 525
   Phe Glu Val Leu Glu
   530
<210> 9
   <211> 517
   <212> PRT
   <213> Lagovirus
<400> 9
   Met Thr Ser Asn Phe Phe Cys Gly Glu Pro Ile Asp Tyr Arg Gly Ile
   1               5                   10                  15
   Thr Ala His Arg Leu Val Gly Ala Glu Pro Arg Pro Pro Val Ser Gly
               20                  25                  30
   Thr Arg Tyr Ala Lys Val Pro Gly Val Pro Asp Glu Tyr Lys Thr Gly
           35                  40                  45
   Tyr Arg Pro Ala Asn Leu Gly Arg Ser Asp Pro Asp Ser Asp Lys Ser
       50                  55                  60
   Leu Met Asn Ile Ala Val Lys Asn Leu Gln Val Tyr Gln Gln Glu Pro
   65                  70                  75                  80
   Lys Leu Asp Lys Val Asp Glu Phe Ile Glu Arg Ala Ala Ala Asp Val
                   85                  90                  95
   Leu Gly Tyr Leu Arg Phe Leu Thr Lys Gly Glu Arg Gln Ala Asn Leu
               100                 105                 110
   Asn Phe Lys Ala Ala Phe Asn Thr Leu Asp Leu Ser Thr Ser Cys Gly
           115                 120                 125
   Pro Phe Val Pro Gly Lys Lys Ile Asp His Val Lys Asp Gly Val Met
       130                 135                 140
   Asp Gln Val Leu Ala Lys His Leu Tyr Lys Cys Trp Ser Val Ala Asn
   145 150 155 160
   Ser Gly Lys Ala Leu His His Ile Tyr Ala Cys Gly Leu Lys Asp Glu
   165 170 175
   Leu Arg Pro Leu Asp Lys Val Lys Glu Gly Lys Lys Arg Leu Leu Trp
   180 185 190
   Gly Cys Asp Val Gly Val Ala Val Cys Ala Ala Ala Val Phe His Asn
   195 200 205
   Ile Cys Tyr Lys Leu Lys Met Val Ala Arg Phe Gly Pro Ile Ala Val
   210 215 220
   Gly Val Asp Met Thr Ser Arg Asp Val Asp Val Ile Ile Asn Asn Leu
   225 230 235 240
   Thr Ser Lys Ala Ser Asp Phe Leu Cys Leu Asp Tyr Ser Lys Trp Asp
   245 250 255
   Ser Thr Met Ser Pro Cys Val Val Arg Leu Ala Ile Asp Ile Leu Ala
   260 265 270
   Asp Cys Cys Glu Gln Thr Glu Leu Thr Lys Ser Val Val Leu Thr Leu
   275 280 285
   Lys Ser His Pro Met Thr Ile Leu Asp Ala Met Ile Val Gln Thr Lys
   290 295 300
   Arg Gly Leu Pro Ser Gly Met Pro Phe Thr Ser Val Ile Asn Ser Ile
   305 310 315 320
   Cys His Trp Leu Leu Trp Ser Ala Ala Val Tyr Lys Ser Cys Ala Glu
   325 330 335
   Ile Gly Leu His Cys Ser Asn Leu Tyr Glu Asp Ala Pro Phe Tyr Thr
   340 345 350
   Tyr Gly Asp Asp Gly Val Tyr Ala Met Thr Pro Met Met Val Ser Leu
   355 360 365
   Leu Pro Ala Ile Ile Glu Asn Leu Arg Asp Tyr Gly Leu Ser Pro Thr
   370 375 380
   Ala Ala Asp Lys Thr Glu Phe Ile Asp Val Cys Pro Leu Asn Lys Ile
   385 390 395 400
   Ser Phe Leu Lys Arg Thr Phe Glu Leu Thr Asp Ile Gly Trp Val Ser
   405 410 415
   Lys Leu Asp Lys Ser Ser Ile Leu Arg Gln Leu Glu Trp Ser Lys Thr
   420 425 430
   Thr Ser Arg His Met Val Ile Glu Glu Thr Tyr Asp Leu Ala Lys Glu
   435 440 445
   Glu Arg Gly Val Gln Leu Glu Glu Leu Gln Val Ala Ala Ala Ala His
   450 455 460
   Gly Gln Glu Phe Phe Asn Phe Val Cys Arg Glu Leu Glu Arg Gln Gln
   465 470 475 480
   Ala Tyr Thr Gln Phe Ser Val Tyr Ser Tyr Asp Ala Ala Arg Lys Ile
   485 490 495
   Leu Ala Asp Arg Lys Arg Val Val Ser Val Val Pro Asp Asp Glu Phe
   500 505 510
   Val Asn Val Met Glu
   515
<210> 10
   <211> 526
   <212> PRT
   <213> Artificial
<220>
   <223> Norovirus RNA polymerase containing His tag
<400> 10
   Met Gly Gly Asp Ser Lys Gly Thr Tyr Cys Gly Ala Pro Ile Leu Gly
   1               5                   10                  15
   Pro Gly Ser Ala Pro Lys Leu Ser Thr Lys Thr Lys Phe Trp Arg Ser
               20                  25                  30
   Ser Thr Thr Pro Leu Pro Pro Gly Thr Tyr Glu Pro Ala Tyr Leu Gly
           35                  40                  45
   Gly Lys Asp Pro Arg Val Lys Gly Gly Pro Ser Leu Gln Gln Val Met
       50                  55                  60
   Arg Asp Gln Leu Lys Pro Phe Thr Glu Pro Arg Gly Lys Pro Pro Lys
   65                  70                  75                  80
   Pro Ser Val Leu Glu Ala Ala Lys Lys Thr Ile Ile Asn Val Leu Glu
                   85                  90                  95
   Gln Thr Ile Asp Pro Pro Glu Lys Trp Ser Phe Thr Gln Ala Cys Ala
           100                 105                 110
   Ser Leu Asp Lys Thr Thr Ser Ser Gly His Pro His His Met Arg Lys
       115                 120                 125
   Asn Asp Cys Trp Asn Gly Glu Ser Phe Thr Gly Lys Leu Ala Asp Gln
   130                 135                 140
   Ala Ser Lys Ala Asn Leu Met Phe Glu Gly Gly Lys Asn Met Thr Pro
   145                 150                 155                 160
   Val Tyr Thr Gly Ala Leu Lys Asp Glu Leu Val Lys Thr Asp Lys Ile
                   165                 170                 175
   Tyr Gly Lys Ile Lys Lys Arg Leu Leu Trp Gly Ser Asp Leu Ala Thr
               180                 185                 190
   Met Ile Arg Cys Ala Arg Ala Phe Gly Gly Leu Met Asp Glu Leu Lys
           195                 200                 205
   Ala His Cys Val Thr Leu Pro Ile Arg Val Gly Met Asn Met Asn Glu
       210                 215                 220
   Asp Gly Pro Ile Ile Phe Glu Arg His Ser Arg Tyr Lys Tyr His Tyr
   225                 230                 235                 240
   Asp Ala Asp Tyr Ser Arg Trp Asp Ser Thr Gln Gln Arg Ala Val Leu
                   245                 250                 255
   Ala Ala Ala Leu Glu Ile Met Val Lys Phe Ser Ser Glu Pro His Leu
               260                 265                 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465                 470                 475                 480
   Pro Met Phe Arg Trp Met Arg Phe Ser Asp Leu Ser Thr Trp Glu Gly
                   485                 490                 495
   Asp Arg Asn Leu Ala Pro Ser Phe Val Asn Glu Asp Gly Val Glu Val
               500                 505                 510
   Asp Lys Leu Ala Ala Ala Leu Glu His His His His His His
           515                 520                 525
<210> 11
   <211> 523
   <212> PRT
   <213> Artificial
<220>
   <223> Sapovirus RNA polymerase containing His tag
<400> 11
   Met Lys His His His His His His Asp Glu Phe Gln Trp Lys Gly Leu
   1               5                   10                  15
   Pro Val Val Lys Ser Gly Leu Asp Val Gly Gly Met Pro Thr Gly Thr
               20                  25                  30
   Arg Tyr His Arg Ser Pro Ala Trp Pro Glu Glu Gln Pro Gly Glu Thr
           35                  40                  45
   His Ala Pro Ala Pro Phe Gly Ala Gly Asp Lys Arg Tyr Thr Phe Ser
   50 55 60
   Gln Thr Glu Met Leu Val Asn Gly Leu Lys Pro Tyr Thr Glu Pro Thr
   65 70 75 80
   Ala Gly Val Pro Pro Gln Leu Leu Ser Arg Ala Val Thr His Val Arg
   85 90 95
   Ser Tyr Ile Glu Thr Ile Ile Gly Thr His Arg Ser Pro Val Leu Thr
   100 105 110
   Tyr His Gln Ala Cys Glu Leu Leu Glu Arg Thr Thr Ser Cys Gly Pro
   115 120 125
   Phe Val Gln Gly Leu Lys Gly Asp Tyr Trp Asp Glu Glu Gln Gln Gln
   130 135 140
   Tyr Thr Gly Val Leu Ala Asn His Leu Glu Gln Ala Trp Asp Lys Ala
   145 150 155 160
   Asn Lys Gly Ile Ala Pro Arg Asn Ala Tyr Lys Leu Ala Leu Lys Asp
   165 170 175
   Glu Leu Arg Pro Ile Glu Lys Asn Lys Ala Gly Lys Arg Arg Leu Leu
   180 185 190
   Trp Gly Cys Asp Ala Ala Thr Thr Leu Ile Ala Thr Ala Ala Phe Lys
   195 200 205
   Ala Val Ala Thr Arg Leu Gln Val Val Thr Pro Met Thr Pro Val Ala
   210 215 220
   Val Gly Ile Asn Met Asp Ser Val Gln Met Gln Val Met Asn Asp Ser
   225 230 235 240
   Leu Lys Gly Gly Val Leu Tyr Cys Leu Asp Tyr Ser Lys Trp Asp Ser
   245 250 255
   Thr Gln Asn Pro Ala Val Thr Ala Ala Ser Leu Ala Ile Leu Glu Arg
   260 265 270
   Phe Ala Glu Pro His Pro Ile Val Ser Cys Ala Ile Glu Ala Leu Ser
   275 280 285
   Ser Pro Ala Glu Gly Tyr Val Asn Asp Ile Lys Phe Val Thr Arg Gly
   290 295 300
   Gly Leu Pro Ser Gly Met Pro Phe Thr Ser Val Val Asn Ser Ile Asn
   305 310 315 320
   His Met Ile Tyr Val Ala Ala Ala Ile Leu Gln Ala Tyr Glu Ser His
   325 330 335
   Asn Val Pro Tyr Thr Gly Asn Val Phe Gln Val Glu Thr Val His Thr
   340 345 350
   Tyr Gly Asp Asp Cys Met Tyr Ser Val Cys Pro Ala Thr Ala Ser Ile
   355 360 365
   Phe His Ala Val Leu Ala Asn Leu Thr Ser Tyr Gly Leu Lys Pro Thr
   370 375 380
   Ala Ala Asp Lys Ser Asp Ala Ile Lys Pro Thr Asn Thr Pro Val Phe
   385 390 395 400
   Leu Lys Arg Thr Phe Thr Gln Thr Pro His Gly Val Arg Ala Leu Leu
   405 410 415
   Asp Ile Thr Ser Ile Thr Arg Gln Phe Tyr Trp Leu Lys Ala Asn Arg
   420 425 430
   Thr Ser Asp Pro Ser Ser Pro Pro Ala Phe Asp Arg Gln Ala Arg Ser
   435 440 445
   Ala Gln Leu Glu Asn Ala Leu Ala Tyr Ala Ser Gln His Gly Pro Val
   450 455 460
   Val Phe Asp Thr Val Arg Gln Ile Ala Ile Lys Thr Ala Gln Gly Glu
   465 470 475 480
   Gly Leu Val Leu Val Asn Thr Asn Tyr Asp Gln Ala Leu Ala Thr Tyr
   485 490 495
   Asn Ala Trp Phe Ile Gly Gly Thr Val Pro Asp Pro Val Gly His Thr
   500 505 510
   Glu Gly Thr His Lys Ile Val Phe Glu Met Glu
   515 520
<210> 12
   <211> 523
   <212> PRT
   <213> Artificial
<220>
   <223> Sapovirus RNA polymerase containing His tag
<400> 12
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
       210                 215                 220
   Ser Val Gln Met Gln Val Met Asn Asp Ser Leu Lys Gly Gly Val Leu
   225                 230                 235                 240
   Tyr Cys Leu Asp Tyr Ser Lys Trp Asp Ser Thr Gln Asn Pro Ala Val
                   245                 250                 255
   Thr Ala Ala Ser Leu Ala Ile Leu Glu Arg Phe Ala Glu Pro His Pro
               260                 265                 270
   Ile Val Ser Cys Ala Ile Glu Ala Leu Ser Ser Pro Ala Glu Gly Tyr
           275                 280                 285
   Val Asn Asp Ile Lys Phe Val Thr Arg Gly Gly Leu Pro Ser Gly Met
       290                 295                 300
   Pro Phe Thr Ser Val Val Asn Ser Ile Asn His Met Ile Tyr Val Ala
   305                 310                 315                 320
   Ala Ala Ile Leu Gln Ala Tyr Glu Ser His Asn Val Pro Tyr Thr Gly
                   325                 330                 335
   Asn Val Phe Gln Val Glu Thr Val His Thr Tyr Gly Asp Asp Cys Met
               340                 345                 350
   Tyr Ser Val Cys Pro Ala Thr Ala Ser Ile Phe His Ala Val Leu Ala
           355                 360                 365
   Asn Leu Thr Ser Tyr Gly Leu Lys Pro Thr Ala Ala Asp Lys Ser Asp
       370                 375                 380
   Ala Ile Lys Pro Thr Asn Thr Pro Val Phe Leu Lys Arg Thr Phe Thr
   385                 390                 395                 400
   Gln Thr Pro His Gly Val Arg Ala Leu Leu Asp Ile Thr Ser Ile Thr
                   405                 410                 415
   Arg Gln Phe Tyr Trp Leu Lys Ala Asn Arg Thr Ser Asp Pro Ser Ser
               420                 425                 430
   Pro Pro Ala Phe Asp Arg Gln Ala Arg Ser Ala Gln Leu Glu Asn Ala
           435                 440                 445
   Leu Ala Tyr Ala Ser Gln His Gly Pro Val Val Phe Asp Thr Val Arg
       450                 455                 460
   Gln Ile Ala Ile Lys Thr Ala Gln Gly Glu Gly Leu Val Leu Val Asn
   465                 470                 475                 480
   Thr Asn Tyr Asp Gln Ala Leu Ala Thr Tyr Asn Ala Trp Phe Ile Gly
                   485                 490                 495
   Gly Thr Val Pro Asp Pro Val Gly His Thr Glu Gly Thr His Lys Ile
               500                 505                 510
   Val Phe Glu Met Glu His His His His His His
           515                 520
<210> 13
   <211> 539
   <212> PRT
   <213> Artificial
<220>
   <223> Vesivirus RNA polymerase containing His tag
<400> 13
   Met Lys Val Thr Thr Gln Lys Tyr Asp Val Thr Lys Pro Asp Ile Ser
   1               5                   10                  15
   Tyr Lys Gly Leu Ile Cys Lys Gln Leu Asp Glu Ile Arg Val Ile Pro
               20                  25                  30
   Lys Gly Thr Arg Leu His Val Ser Pro Ala His Thr Asp Asp Tyr Asp
           35                  40                  45
   Glu Cys Ser His Gln Pro Ala Ser Leu Gly Ser Gly Asp Pro Arg Cys
       50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145                 150                 155                 160
   Ala Thr Gln Gly Ile Gly Leu Pro His Glu Tyr Thr Ile Gly Leu Lys
                   165                 170                 175
   Asp Glu Leu Arg Pro Val Glu Lys Val Gln Glu Gly Lys Arg Arg Met
               180                 185                 190
   Ile Trp Gly Cys Asp Val Gly Val Ala Thr Val Cys Ala Ala Ala Phe
           195                 200                 205
   Lys Gly Val Ser Asp Ala Ile Thr Ala Asn His Gln Tyr Gly Pro Val
       210                 215                 220
   Gln Val Gly Ile Asn Met Asp Gly Pro Ser Val Glu Ala Leu Tyr Gln
   225                 230                 235                 240
   Arg Ile Arg Ser Ala Ala Lys Val Phe Ala Val Asp Tyr Ser Lys Trp
                   245                 250                 255
   Asp Ser Thr Gln Ser Pro Arg Val Ser Ala Ala Ser Ile Asp Ile Leu
               260                 265                 270
   Arg Tyr Phe Ser Asp Arg Ser Pro Ile Val Asp Ser Ala Ala Asn Thr
           275                 280                 285
   Leu Lys Ser Pro Pro Ile Ala Ile Phe Asn Gly Val Ala Val Lys Val
       290                 295                 300
   Thr Ser Gly Leu Pro Ser Gly Met Pro Leu Thr Ser Val Ile Asn Ser
   305                 310                 315                 320
   Leu Asn His Cys Leu Tyr Val Gly Cys Ala Ile Leu Gln Ser Leu Glu
                   325                 330                 335
   Ser Arg Asn Ile Pro Val Thr Trp Asn Leu Phe Ser Thr Phe Asp Met
               340                 345                 350
   Met Thr Tyr Gly Asp Asp Gly Val Tyr Met Phe Pro Met Met Phe Ala
           355                 360                 365
   Ser Val Ser Asp Gln Ile Phe Ala Asn Leu Thr Ala Tyr Gly Leu Lys
       370                 375                 380
   Pro Thr Arg Val Asp Lys Ser Val Gly Ala Ile Glu Pro Ile Asp Pro
   385                 390                 395                 400
   Glu Ser Val Val Phe Leu Lys Arg Thr Ile Thr Arg Thr Pro His Gly
                   405                 410                 415
   Ile Arg Gly Leu Leu Asp Arg Gly Ser Ile Ile Arg Gln Phe Tyr Tyr
               420                 425                 430
   Ile Lys Gly Glu Asn Ser Asp Asp Trp Lys Thr Pro Pro Lys Thr Ile
           435                 440                 445
   Asp Pro Thr Ser Arg Gly Gln Gln Leu Trp Asn Ala Cys Leu Tyr Ala
       450                 455                 460
   Ser Gln His Gly Pro Glu Phe Tyr Asn Lys Val Tyr Arg Leu Ala Glu
   465                 470                 475                 480
   Lys Ala Val Glu Tyr Glu Glu Leu His Phe Glu Pro Pro Ser Tyr His
   485 490 495
   Ser Ala Leu Glu His Tyr Asn Asn Gln Phe Asn Gly Val Asp Thr Arg
   500 505 510
   Ser Asp Gln Ile Asp Ala Ser Val Met Thr Asp Leu His Cys Asp Val
   515 520 525
   Phe Glu Val Leu Glu His His His His His His
   530 535
<210> 14
   <211> 523
   <212> PRT
   <213> Artificial
<220>
   <223> Lagovirus RNA Polymerase containing His tag
<400> 14
   Met Thr Ser Asn Phe Phe Cys Gly Glu Pro Ile Asp Tyr Arg Gly Ile
   1               5                   10                  15
   Thr Ala His Arg Leu Val Gly Ala Glu Pro Arg Pro Pro Val Ser Gly
               20                  25                  30
   Thr Arg Tyr Ala Lys Val Pro Gly Val Pro Asp Glu Tyr Lys Thr Gly
           35                  40                  45
   Tyr Arg Pro Ala Asn Leu Gly Arg Ser Asp Pro Asp Ser Asp Lys Ser
       50                  55                  60
   Leu Met Asn Ile Ala Val Lys Asn Leu Gln Val Tyr Gln Gln Glu Pro
   65                  70                  75                      80
   Lys Leu Asp Lys Val Asp Glu Phe Ile Glu Arg Ala Ala Ala Asp Val
                   85                  90                  95
   Leu Gly Tyr Leu Arg Phe Leu Thr Lys Gly Glu Arg Gln Ala Asn Leu
               100                 105                 110
   Asn Phe Lys Ala Ala Phe Asn Thr Leu Asp Leu Ser Thr Ser Cys Gly
           115                 120                 125
   Pro Phe Val Pro Gly Lys Lys Ile Asp His Val Lys Asp Gly Val Met
       130                 135                 140
   Asp Gln Val Leu Ala Lys His Leu Tyr Lys Cys Trp Ser Val Ala Asn
   145                 150                 155                     160
   Ser Gly Lys Ala Leu His His Ile Tyr Ala Cys Gly Leu Lys Asp Glu
   165 170 175
   Leu Arg Pro Leu Asp Lys Val Lys Glu Gly Lys Lys Arg Leu Leu Trp
   180 185 190
   Gly Cys Asp Val Gly Val Ala Val Cys Ala Ala Ala Val Phe His Asn
   195 200 205
   Ile Cys Tyr Lys Leu Lys Met Val Ala Arg Phe Gly Pro Ile Ala Val
   210 215 220
   Gly Val Asp Met Thr Ser Arg Asp Val Asp Val Ile Ile Asn Asn Leu
   225 230 235 240
   Thr Ser Lys Ala Ser Asp Phe Leu Cys Leu Asp Tyr Ser Lys Trp Asp
   245 250 255
   Ser Thr Met Ser Pro Cys Val Val Arg Leu Ala Ile Asp Ile Leu Ala
   260 265 270
   Asp Cys Cys Glu Gln Thr Glu Leu Thr Lys Ser Val Val Leu Thr Leu
   275 280 285
   Lys Ser His Pro Met Thr Ile Leu Asp Ala Met Ile Val Gln Thr Lys
   290 295 300
   Arg Gly Leu Pro Ser Gly Met Pro Phe Thr Ser Val Ile Asn Ser Ile
   305 310 315 320
   Cys His Trp Leu Leu Trp Ser Ala Ala Val Tyr Lys Ser Cys Ala Glu
   325 330 335
   Ile Gly Leu His Cys Ser Asn Leu Tyr Glu Asp Ala Pro Phe Tyr Thr
   340 345 350
   Tyr Gly Asp Asp Gly Val Tyr Ala Met Thr Pro Met Met Val Ser Leu
   355 360 365
   Leu Pro Ala Ile Ile Glu Asn Leu Arg Asp Tyr Gly Leu Ser Pro Thr
   370 375 380
   Ala Ala Asp Lys Thr Glu Phe Ile Asp Val Cys Pro Leu Asn Lys Ile
   385 390 395 400
   Ser Phe Leu Lys Arg Thr Phe Glu Leu Thr Asp Ile Gly Trp Val Ser
   405 410 415
   Lys Leu Asp Lys Ser Ser Ile Leu Arg Gln Leu Glu Trp Ser Lys Thr
               420                 425                 430
   Thr Ser Arg His Met Val Ile Glu Glu Thr Tyr Asp Leu Ala Lys Glu
           435                 440                 445
   Glu Arg Gly Val Gln Leu Glu Glu Leu Gln Val Ala Ala Ala Ala His
       450                 455                 460
   Gly Gln Glu Phe Phe Asn Phe Val Cys Arg Glu Leu Glu Arg Gln Gln
   465                 470                 475                 480
   Ala Tyr Thr Gln Phe Ser Val Tyr Ser Tyr Asp Ala Ala Arg Lys Ile
                   485                 490                 495
   Leu Ala Asp Arg Lys Arg Val Val Ser Val Val Pro Asp Asp Glu Phe
               500                 505                 510
   Val Asn Val Met Glu His His His His His His
           515                 520
<210> 15
   <211> 24
   <212> RNA
   <213> Artificial
<220>
   <223> ssRNA template
<400> 15
   auaccuagaa ucugaccaac cccc 24
<210> 16
   <211> 24
   <212> DNA
   <213> Artificial
<220>
   <223> ssDNA template
<400> 16
   atacctagaa tctgaccaac cccc 24
<210> 17
   <211> 24
   <212> DNA
   <213> Artificial
<220>
   <223> ssDNA template having 5 rC at the 3'-end
<220>
   <221> misc_feature
   <222> (1)..(19)
   <223> deoxyribonucleotides
<220>
   <221> misc_feature
   <222> (20)..(24)
   <223> ribonucleotides
<400> 17
   atacctagaa tctgaccaac cccc 24

## Description

The present invention relates to a method for polymerising a complementary RNA strand on a single-stranded polynucleotide template comprising the step of irradiating a composition containing said template and an RNA polymerase of a virus of the *Caliciviridae* family under RNA polymerisation conditions in the presence or absence of a primer hybridised to the template, with an effective amount of microwave energy. Further subject matter of the invention relates to a method for transferring one or more ribonucleotides to the 3' end of a single-stranded polynucleotide template comprising the step of irradiating a composition containing an RNA polymerase of a virus of the *Caliciviridae* family in the presence of rATP or rGTP or rUTP or rCTP or a modified or labelled analogue thereof with an effective amount of microwave energy.

RNA-dependent RNA polymerases (in the following denoted as "RNA polymerases") of viruses of the *Caliciviridae* family are known to polymerise a complementary RNA strand on an RNA template in the presence or absence of a primer (see WO-A-2007/12329). Using such RNA polymerases, a typical amplification of RNA at usual temperature conditions takes approximately 2 hours.

US 5,350,686 generally claims microwave acceleration of enzyme-catalysed modifications of macromolecules, but factually shows only successful microwave-assisted reactions in the case of restriction enzymes.

US 7,537,917 describes a microwave-assisted PCR amplification of DNA. However, no data whatsoever are presented that factually show a successful DNA polymerisation by DNA polymerases under microwave irradiation.

In particular with regard to DNA amplification using PCR, only the heating (i.e denaturation) step (and not the polymerisation step) within the amplification cycles has been reported to be a step suitable for microwave application; see Fermér et al. (2003) European J. Pharm. Sci. 18, 129-132.

WO2009/150156 discloses a method for synthesizing modified RNA on a ssRNA template employing an RNA dependent RNA polymerase in the presence of at least one ribonucleoside triphosphate having a chemical modification to yield in chemically modified double-stranded RNA. The RNA-dependent RNA polymerase is of a virus from the Caliciviridae family e.g. norovirus, sapovirus, vesivirus or lagovirus. The nucleoside-triphosphates added for the extension reaction can be deoxyribonucleosides or ribonucleosides. RNA-polymerases of the calicivirus family are capable of synthesizing complementary strands by elongation of a corresponding primer and by de novo synthesis of a complementary strand in the absence of a primer

WO2007/012329 discloses the conditions for a RNA-polymerase of the Caliciviridae family requiring a primer or not in correlation to the sequence of the template and does not disclose that said RNA-polymerization reaction can be driven by microwave energy.

EP1840226 discloses PCR amplification under microwave irradiation.

WO93/21344 discloses microwave irradiation of DNA or RNA in restriction endonuclease digestion.

The technical problem underlying the present invention is to provide an improved method of RNA polymerisation on polynucleotide templates.

The solution to the above technical problem is provided by the embodiments of the present invention as described herein and characterised in the claims.

In particular, it has been surprisingly found that the complex reaction of polymerising a complementary RNA strand on a single-stranded polynucleotide template (RNA, DNA, mixed RNA/DNA or mixtures thereof) is feasible and greatly enhanced by microwave irradiation when using RNA polymerases of viruses of the *Caliciviridae* family but not other RNA polymerases studied in the experiments leading to the present invention.

Thus, the present invention relates to a method for polymerising a complementary RNA strand on a single-stranded polynucleotide template comprising irradiating a composition containing said template and an RNA polymerase of a virus of the *Caliciviridae* family under RNA polymerisation conditions in the presence or absence of a primer hybridised to the template, to an effective amount of microwave energy.

RNA polymerases of the caliciviruses have (as have other viral RNA-dependent RNA polymerases such as RNA polymerase from poliovirus or Hepatitis C Virus (HCV) which, however, are not applicable to the inventive method; cf. the Examples described below) the following structural features in common: RNA polymerases of the viruses of the *Caliciviridae* family have a "right hand conformation" and the amino acid sequence of said RNA polymerases comprises a conserved arrangement of the following sequence motifs:
a. XXDYS (SEQ ID NO: 1)
b. GXPSG (SEQ ID NO: 2)
c. YGDD (SEQ ID NO: 3)
d. XXYGL (SEQ ID NO: 4)
e. XXXXFLXRXX (SEQ ID NO: 5)
with the following meanings:
D: aspartate
Y: tyrosine
S: serine
G: glycine
P: proline
L: leucine
F: phenylalanine
R: arginine
X: any amino acid.

The so-called "right hand conformation" as used herein means that the tertiary structure (conformation) of the RNA polymerase folds like a right hand with finger, palm and thumb, as observed in most template-dependent polymerases.

The sequence motif "XXDYS" (SEQ ID NO: 1) is the so-called A-motif. The A-motif is usually responsible for the discrimination between ribonucleosides and deoxyribonucleosides. The motif "GXPSG" (SEQ ID NO: 2) is the so-called B-motif. The B-motif is conserved within all representatives of the RNA polymerases of the *Caliciviridae* family. The motif "YGDD" (C-motif, SEQ ID NO: 3) represents the active site of the enzyme. This motif, in particular the first aspartate residue (in bold, YGDD) plays an important role in the coordination of the metal ions during the Mg²⁺/Mn²⁺ dependent catalysis. The motif "XXYGL" (SEQ ID NO: 4) is the so-called D-motif. The D-motif is a feature of template-dependent polymerases. Finally, the "XXXXFLXRXX" motif (E-motif, SEQ ID NO: 5) is a feature of RNA-dependent RNA polymerases which discriminates them from (exclusively) DNA-dependent RNA polymerases.

Preferably, the RNA polymerase is an RNA polymerase of a human and/or non-human pathogenic calicivirus. Especially preferred is an RNA polymerase of a norovirus, sapovirus, vesivirus or lagovirus, for example, an RNA polymerase of the norovirus strain HuCV/NUDresden174/1997/GE (GenBank Acc. No AY741811) or an RNA polymerase of the sapovirus strain pJG-Sap01 (GenBank Acc. No AY694184) or an RNA polymerase of the vesivirus strain FCV/Dresden/2006/GE (GenBank Acc. No DQ424892) or an RNA polymerase of the lagovirus strain pJG-RHDV-DD06 (GenBank Acc. No. EF363035.1).

According to especially preferred embodiments of the invention the RNA polymerase is a protein comprising (or having) an amino acid sequence according SEQ ID NO: 6 (norovirus RNA polymerase), SEQ ID NO: 7 (sapovirus RNA polymerase), SEQ ID NO: 8 (vesivirus RNA polymerase) or SEQ ID NO: 9 (lagovirus RNA polymerase). The person skilled in the art is readily capable of preparing such RNA polymerases, for example by recombinant expression using suitable expression vectors and host organisms (cf. WO-A-2007/012329). To facilitate purification of the RNA polymerase after recombinant expression, it is preferred that the RNA polymerase is expressed with a suitable tag (for example GST or (His)₆-tag) at the N- or C-terminus of the corresponding sequence. For example, a histidine tag allows the purification of the protein by affinity chromatography over a nickel or cobalt column in a known fashion. Examples of embodiments of RNA polymerases fused to a histidine tag are the proteins comprising (or having) an amino acid sequence according to SEQ ID NO: 10, SEQ ID NO: 11, SEQ ID NO: 12, SEQ ID NO: 13 or SEQ ID NO: 14. SEQ NO: NO: 10 corresponds to an RNA polymerase of a norovirus having a histidine tag. SEQ ID NO: 11 and SEQ ID NO: 12 correspond to amino acid sequences of an RNA polymerase of a sapovirus having a histidine tag. SEQ ID NO: 13 corresponds to the amino acid sequence of an RNA polymerase of a vesirius having a histidine tag. SEQ ID NO: 14 corresponds to the amino acid sequence of an RNA polymerase of a lagovirus having a histidine tag.

The above-defined RNA polymerase of a calicivirus is capable of synthesizing a complementary RNA strand on a polynucleotide strand of ribonucleotides (i.e. the polynucleotide template consists of or comprises RNA) or deoxyribonucleotides (i.e. the polynucleotide template consists of or comprises DNA). Accordingly, the polynucleotide template may be single-stranded RNA, single-stranded DNA, single-stranded mixed DNA/RNA or a mixture of such species. If the polynucleotide template comprises or consists of DNA, the irradiation with microwaves must be carried out in the presence of a modified GTP, preferably a 2'.modified GTP such as 2'-fluoro-GTP, or α-thio-GTP. The RNA polymerase of a calicivirus synthesises a complementary RNA strand on a single-stranded polynucleotide both by elongation of a primer having a complementary sequence to a partial sequence of the template DNA and by *de novo* synthesis of a complementary strand in the absence of a primer. However, if the polynucleotide template containing deoxyribonucleotides has a deoxyribonucleotide at its 3'-end (i.e. the last nucleotide at the 3'-end of the single-stranded template) which is not a deoxy-C nucleoteotide (i.e. has a deoxy-T, deoxy-A or deoxy-G nucleotide at its 3'-end), the RNA polymerase of a calicivirus useful in the present invention requires the presence of a primer hybridised to the template for synthesis of an RNA strand complementary to the template. If the polynucleotide template as defined herein consists of or contains one or more deoxyribonucleotides at its 3'-end (i.e. the 3'-end of the template is a DNA segment or only the last nucleotide is a deoxyribonucleotide), it is preferred that the last deoxyribonucleotide at the 3'-end of the template is a dC, more preferred at least the last two, three, four or five deoxyribonucleotides at the 3'-end of the template are dC nucleotides for increasing the efficiency of *de novo* initiation of RNA synthesis in the absence of a primer.

In case of templates consisting of RNA or of templates in which the region at the 3'-end is composed of ribonucleotides, the polymerisation in the inventive microwave irradiation method requires the presence of a corresponding primer, if the template is polyadenylated, polyguanylated or polyuridylated. If the template is polycytidylated, RNA synthesis by the calicivirus RNA polymerase is also possible without a primer. In this embodiment it is preferred that elevated rGTP levels (i.e. a surplus of GTP such as, e.g., 2x, 3x, 4x or 5x compared to the other required rNTPs) are present.

The primer, if desired or required, respectively, may be a sequence specific (heteropolymeric) DNA or RNA or mixed DNA/RNA primer or may be a random primer (DNA or RNA or mixed DNA/RNA) or may be a homopolymeric primer such as an oligo-dT-primer or an oligo-U-Primer. The length of the primer is not critical for carrying out the inventive method, but usually oligonucleotide primers having a length of, for example, about 5 to about 25 nt, more preferred about 10 to 20 nt, most preferred about 15 to about 20 nt, are especially useful. More details of the characteristic features of the calicivirus RNA polymerase can be found in WO-A-2007/012329. In contrast to other RNA-dependent RNA polymerases, e.g. RNA-dependent RNA polymerases such as replicases of the Qß type, the RNA polymerases of the caliciviruses do not require primers having a specific recognition sequence for the polymerase to start RNA synthesis. Thus, a "primer" as used herein is typically a primer not having such recognition sequences, in particular, of RNA polymerases. Furthermore, the calicivirus RNA polymerases are different from usual DNA-dependent RNA polymerases such as T7 RNA polymerase in that they do not require specific promoter sequences to be present in the template.

The single-stranded polynucleotide template may comprise at least a sequence segment of deoxyribonucleotides, i.e. at least a segment of ssDNA, e.g. at least a segment of DNA at the 3'-end of the template. A "segment" in this context means at least 2 or more consecutive deoxyribonucleotides. For example, the polynucleotide template according to the invention may be a single-stranded molecule starting at its 5'-end with ribonucleotides, followed by a "middle" region of deoxyribonucleotides (DNA) and ends (at the 3'-end) again with ribonucleotides. Other examples are species of 5'-RNA-DNA-3' or 5'-DNA-RNA-3' or any other polynucleotides having RNA and DNA sequences. Further examples of single-stranded polynucleotide templates according to the invention include species of predominantly ssDNA, but having one to multiple (such as 1, 2, 3, 4, 5, 6, 7, 8, 9 or 10) ribonucleotides at one or both of the 5'-end and/or 3'-end, preferably at the 3'-end. Alternatively, templates of use according to the invention may be predominantly ssRNA, but having one to multiple (such as 1, 2, 3, 4, 5, 6, 7, 8, 9 or 10) deoxyribonucleotides at one or both of the 5'-end and/or 3'-end, preferably at the 3'-end. Of course, the single-stranded polynucleotide template according to the invention may also consist exclusively of ssDNA or ssRNA.

As mentioned before, polynucleotide templates of the invention having a dA, dT or dG residue at the 3'-end normally require a primer for synthesis of a complementary RNA strand by the RNA polymerase. However, even such polynucleotide sequences not having a C nucleotides at the 3'-end can be efficiently transcribed into RNA by the inventive method without the need of a primer: especially in this case, but not limited thereto, the method may be carried with an initial step of irradiating a composition containing the single-stranded polynucleotide template and the RNA polymerase of a calicivirus as defined above in the presence of rCTP as the only nucleotide with an efficient amount of microwave energy under conditions such that said RNA polymerase adds at least one rC (or more such as 2, 3, 4 or 5 rC) nucleotide to the 3'-end of the template. Thereafter, the thus-produced template having one or more C ribonucleotides at the 3'-end can be introduced to the microwave-driven RNA synthesis by the RNA polymerase as defined above such that said RNA polymerase synthesizes a complementary RNA strand, which step may be carried out in the absence of a primer. It is to be understood, however, that also a primer may be used in this embodiment, for example, if needed to introduce a chosen sequence into the RNA strand to be produced by the RNA polymerase or for other purposes.

In view of the terminal transferase activity of the RNA polymerases of caliciviruses, the present invention provides also a method for transferring one or more ribonucleotides to the 3' end of a single-stranded polynucleotide template as defined above comprising the step of irradiating a composition containing an RNA polymerase of a virus of the *Caliciviridae* family as defined above in the presence of rATP, rGTP, rUTP or rCTP or a modified or labelled analogue thereof, with an efficient amount of microwave energy.

According to a preferred embodiment of the method of the invention, the double-stranded molecule produced by the RNA polymerase under microwave irradiation is separated into single strands resulting in an ssRNA and the template. This step may be carried out by heat or chemical denaturation or enzymatically, e.g. by an enzyme capable of separating single-stranded polynucleotides into single-stranded ones such as a helicase. However, according to especially preferred embodiments of the present invention this and other separation steps of double-stranded polynucleotides produced by the RNA polymerase under microwave irradiation as defined herein is carried out by the same enzyme, i.e. the RNA polymerase itself, which step is preferable also carried out under irradiation with microwaves. This step makes beneficial use of the strand-displacement activity of the RNA polymerases of caliciviruses.

It is further preferred that the single strands obtained as outlined above are again (or further) incubated with the RNA polymerase of a calicivurs as defined herein under microwave irradiation and under conditions such that the RNA polymerase synthesizes an RNA strand complementary to each of said single strands. It is evident that the steps of RNA synthesis and strand separation can be repeated one or more times, e.g. about 3 to about 40, preferably about 5 to about 30, more preferably about 10 to about 20 times.. According to a further preferred embodiment, the method of the invention comprises a final RNA synthesis step. In particular in cases of repeated cycling of strand separation and RNA synthesis, this method leads to the production of almost pure dsRNA, even if the original template contained a segment (one or more) of DNA or consisted of DNA.

As already outlined above, any further strand separation step may be carried out by heat or chemical denaturation or enzymatically, e.g. by an enzyme capable of separating single-stranded polynucleotides into single-stranded ones such as a helicase. More preferably, however, also the further strand separation steps are carried out by the RNA polymerase of a calicivirus. Therefore, it is evident that the preferred method according to the invention comprising several to a multitude of strand separation and RNA synthesis steps may be carried out in a single batch reaction (especially when using templates that do not require a primer for RNA synthesis by the RNA polymerase as defined herein) requiring only one microwave irradiation of a reaction mixture containing the template, an RNA polymerase of a calicivirus, appropriate buffer (see below) and rNTPs (i.e. rATP, rUTP, rCTP and rGTP, or modified or labelled rNTPs as further outlined below).

According to the present invention, the term "RNA polymerisation conditions" means the conditions, in particular relating to buffer, salt and metal ion (if applicable) conditions that allow the RNA polymerase to synthesise an RNA strand complementary to a template strand. Appropriate buffer, salt, metal ion, reducing agent (if applicable) and other conditions of RNA polymerases are known to the skilled person; see, e.g., WO-A-2007/012329. Thus, the polynucleotide template is typically used in amounts of, e.g. 1 microgram to 4 microgram per 50 microliter reaction volume. The concentration of the ribonucleoside triphosphates (including optional modified or labelled ribonucleoside trisphosphate(s) as further outlined below) is preferably in the range of from 0.1 micromol/l to 1 micromol/l, for example 0.4 micromol/l. The concentration of the RNA polymerase may be for example 1 micromol/l to 10 micromol/l.

Typical buffer conditions are 10 to 80 mM, more preferred 20 to 50 mM HEPES, pH 7.0 to 8.0, 1 to 5 mM, for example 3 mM magnesium acetate, magnesium chloride, manganese acetate or manganese chloride and 1 to 5 mM of a reducing agent, for example DTT.

A typical stop solution contains 2 to 10 mM, preferably 4 to 8 mM ammonium acetate, and 50 to 200 mM, for example 150 mM EDTA.

The length and origin of the single-stranded polynucleotide, e.g. an ssRNA template, is generally not critical. The template may have a naturally occurring or artificial sequence, and the template may be chemically synthesized or derived from diverse sources such as total RNA, mRNA or genomic DNA from eukaryotic, prokaryotic or viral origin, plasmid DNA, cDNA, bacmids or any other sources of RNA and DNA. Double-stranded DNA or RNA needs to be separated into ssDNA or ssRNA, respectively, by heat or microwave irradiation or chemical denaturation prior to serving as a template in the methods of the invention.

The method of the present invention is particularly useful for employing RNA templates to either provide double-stranded RNA products and/or to amplify the ssRNA templates. Particularly preferred embodiments of the invention relate to the provision of short RNA molecules for gene silencing applications, either by antisense technology or RNA interference, also for antisense directed against defined sequences of microRNA or noncoding RNA with the aim to inhibit microRNA-driven RNA interference (antagomirs).

For such applications, the template (preferably RNA) to be used in the method of the present invention has typically a length of 8 to 45 nucleotides such as of 15 to 30 nucleotides, preferably of 21 to 28 nucleotides, more preferably of 21 to 23 nucleotides. The molecules of the latter length are particularly useful for siRNA applications.

It is further contemplated that the RNA polymerase employs modified ribonucleotides during RNA synthesis (including terminal transferase activity) under microwave irradiation (besides the optionally present modified GTP (such as 2'-fluoro-GTP or α-thio-GTP) as defined above). For example, the modification may be a label for detecting the double-stranded RNA synthesis product of the RNA polymerase. Alternatively, also the labelling may carried out for detection of the ssRNA product obtained after strand separation. Labels of use in the present invention comprise fluorophores (such fluoresceine), radioactive groups (e.g. ³²P-labelled ribonucleotides) and partners of specific binding pairs such as biotinylated rNTPs.

In certain embodiments of the invention, the at least one modified ribonucleotide to be incorporated by the RNA polymerase activity into the complementary strand may have a chemical modification (one or more of them) at the ribose, phosphate and/or base moiety. With respect to molecules having an increased stability, especially with respect to RNA degrading enzymes, modifications at the backbone, i.e. the ribose and/or phosphate moieties, are especially preferred.

The chemically modified RNA products of the methods of the present invention preferably have an increased stability as compared to the non-modified ss- or dsRNA analogues.

Preferred examples of ribose-modified ribonucleotides are analogues wherein the 2'-OH group is replaced by a group selected from H, OR, R, halo, SH, SR, NH₂, NHR, NR₂ or CN with R being C₁-C₆ alkyl, alkenyl or alkynyl and halo being F, Cl, Br or I. It is clear in the context of the present invention, that the term "modified ribonucleoside triphosphate" or "modified ribonucleotide" also includes 2'-deoxy derivatives which may at several instances also be termed "deoxynucleotides".

Typical examples of such ribonucleotide analogues with a modified ribose at the 2' position include 5-aminoallyl-uridine, 2'-amino-2'-deoxy-uridine, 2'-azido-2'-deoxy-uridine, 2'-fluoro-2'-deoxy-guanosine and 2'-O-methyl-5-methyl-uridine.

Examples of ribonucleotides leading to a phosphate backbone modification in the desired double-stranded product are phosphothioate analogues.

According to the present invention, the at least one modified ribonucleotide may also be selected from analogues having a chemical modification at the base moiety. Examples of such analogues include, 6-aza-uridine, 8-aza-adenosine, 5-bromo-uridine, 7-deaza-adenosine, 7-deaza-guanosine, N⁶-methyl-adenosine, 5-methyl-cytidine, pseudo-uridine, and 4-thio-uridine.

The above and other chemically modified ribonucleoside triphosphates are commercially available, for example from Sigma-Aldrich Chemie GmbH, Munich, Germany or Trilink technologies, USA

It is to be understood that also the polynucleotide template may contain one or more modified or labelled nucleotides as outlined above and/or further known in the art.

Short templates (e.g. as described above) are usually prepared by chemical synthesis. Other methods for providing the single-stranded polynucleotide templates include enzymatic manipulations, for example reverse transcription of RNA and subsequent degradation of the RNA strand, cutting of larger dsDNA molecules by restriction enzyme(s) and subsequent strand separation by heat or chemical denaturation to form ssDNA, preparation of total cellular RNA, preparation of mRNA and so on.

Preferred reaction volumes range from 20 to 200 microliter, preferably 50 to 100 microliter. Typically, the buffer conditions and other conditions as outlined above are provided by mixing appropriate stock solutions (usually 5x or 10x concentrated), adding the RNA polymerase, the template and double distilled or deionised water (which has been preferably made RNAse and/or DNAse free prior to use) to the desired final reaction volume.

The term "effective amount of microwave energy" is the amount of microwave energy required for the RNA polymerisation of a complementary strand on a single-stranded polynucleotide template or to transfer at least one ribonucleotide to the 3'-end of a single-stranded polynucleotide, respectively, using an RNA polymerase of a calicivurs. The concrete amount of microwave energy for a given template may be determined by the skilled person using routine experimentation and depends particularly on the type and length of the template. As used herein the terms "microwave energy", "microwave irradiation" or "irradiation with microwaves" or simply "microwaves" are used synonymously and relate to the part of the electromagnetic spectrum comprising wavelengths of about 0.3 to 30 cm, corresponding to a frequency of 1 to 100 gigahertz, which is found between the radio and the infra-red regions of the electromagnetic spectrum. The amount of electromagnetic energy absorbed by a living organism is determined by the dielectric properties of the tissues, cells, and biological molecules.

The generation of the microwave energy for the purposes of the present invention is not critical and can be by any means known to the art. For example, suitable means for applying microwave radiation to reaction compositions according to the invention are microwave ovens which are commercially available from numerous suppliers and routinely form part of the standard equipment in most biological laboratories. Such microwave ovens typically have maximum power levels of from about 500 W to about 1000 W. Even the smallest ovens provide ample levels of microwave irradiation for use in this invention and accordingly, it will be convenient to use lower power settings on ovens in which the output power is adjustable. Thus, according to preferred embodiments of the inventive methods disclosed herein, the composition is irradiated with microwaves having a frequency of from about 1500 MHz to about 3500 MHz and having a power of from about 50 to about 1000 W.

According to preferred embodiments of this invention, lower power settings are also used to time-distribute the applied power over a longer time interval and minimize the potential for localized energy uptake and resulting molecular damage. In an especially preferred embodiment, microwave power is applied to the sample over a series of intervals, with "rest" intervals, in which microwave power is not applied to the sample. Power application intervals and rest intervals will usually range from 1 to 60 seconds each, with power application intervals of from 15 to 60 seconds and rest intervals from 0.5 to 5 seconds being preferred. Most preferably, power will be applied for intervals of about 45 seconds, separated by rest intervals of 1 to 2 seconds.

However, especially depending on the length of the single-stranded polynucleotide template, the irradiation step may be carried out in a single application (interval) of microwave energy of a time period of 1 s to 5 min, more preferably 3 s to 120 s. The latter short time periods are especially useful when templates of shorter length (such as templates for preparing short dsRNAs such as siRNAs) are employed. In particular with respect to the preparation of dsRNA from ssRNA templates, it has been shown according to the invention that the method as described herein enables substantially shorter reaction times compared to incubation times known in the art.

The use of microwave irradiation for providing double-stranded polynucleotide products by RNA polymerases of the caliciviruses is particularly beneficial in the context of preparing double-stranded RNA. As mentioned before, such reactions have hitherto been carried out using longer incubation times at 30 to 42°C, and heat can used to separate double-stranded RNA products. However, under such circumstances the thus-produced dsRNA tends to degrade such that the production of pure dsRNA having the desired length is often difficult to achieve.

In contrast thereto, the use of microwave energy according to the present invention accelerates the polymerization reaction by the RNA polymerase such that the problem of RNA degradation is avoided.

It is to be understood that in a preferred mode of practicing this invention, the above efforts to distribute the applied power over time are to be taken in addition to using "power" settings of the apparatus below maximum. In fact, many commercial microwave ovens maintain a constant magnetron output power at 650-900 W and modulate applied power by varying the duty cycle of the magnetron, a setting of "1" corresponding to a 10% duty cycle and a setting of "9" corresponding to a 90% duty cycle. Most preferably, settings of from 1 to 8, i.e. a magnetron duty cycle of from 10% to 80% at an output power of 700 to 800 W, will be used. That aggregate output energy, corresponding to from about 70 watt-seconds to 35,000 watt-seconds, preferably from 3000 to 3500 watt-seconds per interval, will be applied in the intervals or a single interval, as described above.

The Figures show:

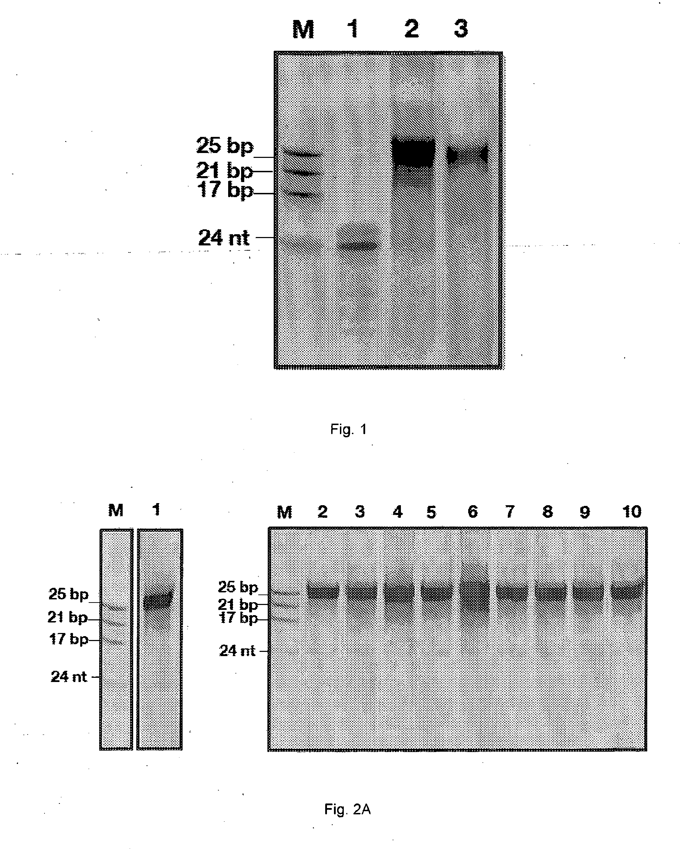

- Fig. 1: shows a photograph of an ethidium bromide-stained native 20% polyacrylamide gel after electrophoretic separation of reactions demonstrating the microwave-driven primer-independent *de novo* initiation of RNA synthesis and generation of a double-stranded RNA by a RNA polymerase of a sapovirus. Lane 1: RNA template. Lane 2: reaction mix of template and RNA polymerase of a sapovirus after microwave irradiation (800 W for 60 s) leads to a band co-migrating with a 25 bp dsRNA marker. Lane 3: product of lane 2 after S1 nuclease treatment. The 25 bp product is not digested with S1 nuclease indicating the double-stranded nature of the product. M: RNA marker corresponding to dsRNA 17 bp, 21 bp and 25 bp and to a ssRNA of 24 nt as indicated.
- Fig. 2A: shows photographs of ethidium bromide-stained native 20% polyacrylamide gels after electrophoretic separation of reactions used to characterize the energy conditions for microwave-driven primer-independent *de novo* initiation of RNA synthesis and generation of a double-stranded RNA by a sapovirus RNA polymerase. The sapovirus RNA polymerase was incubated with a 24 nt ssRNA template under microwave irradiation for 60 s at 80 W (lane 1), 160 W (lane 2), 240 W (lane 3), 320 W (lane 4), 400 W (lane 5), 480 W (lane 6), 560 W (lane 7), 640 W (lane 8), 720 W (lane 9) and 800 W (lane 10), respectively. Lanes 1 to 10 each show a product co-migrating with a 25 bp dsRNA marker. M: RNA marker corresponding to dsRNA of 17 bp, 21 bp and 25 bp, respectively, and to a ssRNA of 24 nt, as indicated.

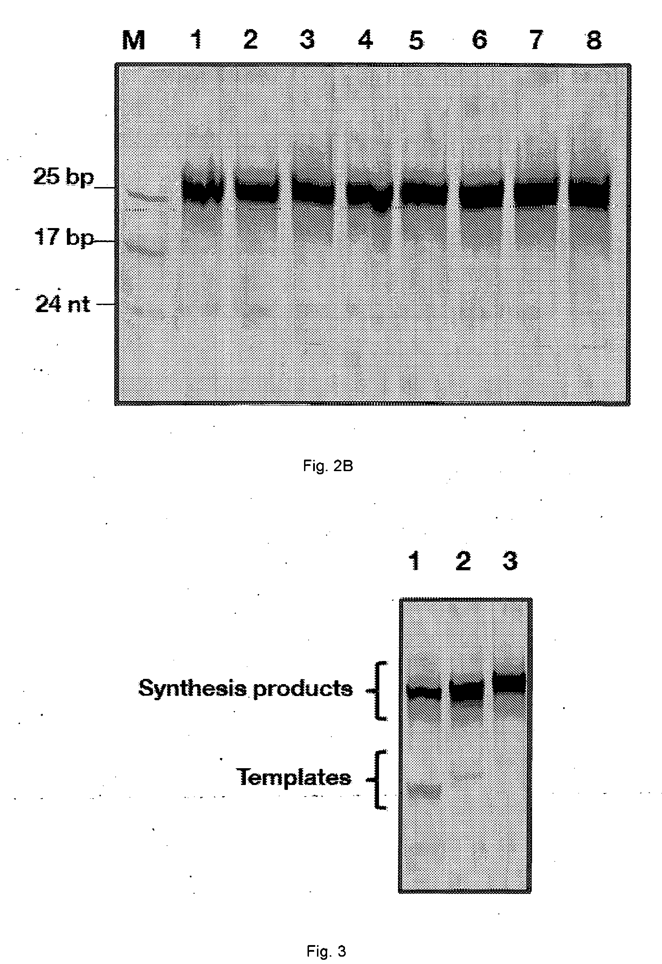

- Fig. 2B: shows a photograph of an ethidium bromide-stained native 20% polyacrylamide gel after electrophoretic separation of reactions used to characterize the irradiation time needed for microwave-driven primer-independent *de novo* initiation of RNA synthesis and generation of a double-stranded RNA by a sapovirus RNA polymerase. The sapovirus RNA polymerase was incubated with a 24 nt ssRNA template under microwave irradiation at 80 W for 60 s (lane 1), 30 s (lane 2), 15 s (lane 3) and 5 s (lane 4), respectively, or at 800 W for 60 s (lane 5), 30 s (lane 6), 15 s (lane 7) and 5 s (lane 8), respectively. Each of lanes 1 to 8 shows a product co-migrating with a 25 bp dsRNA marker. M: RNA marker corresponding to dsRNA of 17 bp and 25 bp, respectively and to a ssRNA of 24 nt, as indicated.
- Fig. 3: shows a photograph of an ethidium bromide-stained native 20% polyacrylamide gel after electrophoretic separation of reactions demonstrating that a sapovirus RNA polymerase initiates RNA synthesis on DNA templates de *novo* in a primer-independent manner and incorporates 2'-fluoro-GMP leading to a double-stranded DNA/RNA product when the reaction is exposed to microwave irradiation. The sapovirus RNA polymerase was incubated with an ssDNA template of 24 nt having 5 dC at the 3'-end (lane 1) or an ssDNA template of the same sequence but having 5 rC at the 3'-end (lane 2) under microwave irradiation at 800 W for 60 s in the presence of rATP, rCTP, rUTP and 2'-fuoro-GTP. A reaction containing the sapovirus RNA polymerase and an ssRNA template of the same sequence as the DNA templates irradiated with microwaves under the same conditions as before and in the presence of rATP, rCTP, rUTP and rGTP served as a control (lane 3). Double-stranded synthesis products and single-stranded templates are indicated.

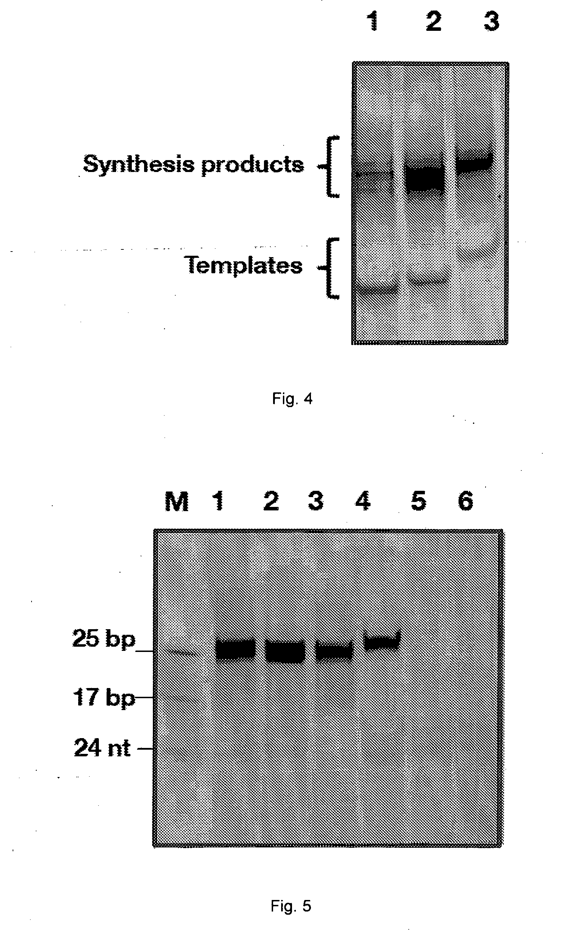

- Fig. 4: shows a photograph of an ethidium bromide-stained native 20% polyacrylamide gel after electrophoretic separation of reactions demonstrating that a sapovirus RNA polymerase initiates RNA synthesis on DNA templates *de novo* in a primer-independent manner and incorporates α-thio-GMP leading to a double-stranded DNA/RNA product when the reaction is exposed to microwave irradiation. The sapovirus RNA polymerase was incubated with an ssDNA template of 24 nt having 5 dC at the 3'-end (lane 1) or an ssDNA template of the same sequence but having 5 rC at the 3'-end (lane 2) under microwave irradiation at 800 W for 60 s in the presence of rATP, rCTP, rUTP and α-thio-GTP. A reaction containing the sapovirus RNA polymerase and an ssRNA template of the same sequence as the DNA templates irradiated with microwaves under the same conditions as before and in the presence of rATP, rCTP, rUTP and rGTP served as a control (lane 3). Double-stranded synthesis products and single-stranded templates are indicated.
- Fig. 5: shows a photograph of an ethidium bromide-stained native 20% polyacrylamide gel after electrophoretic separation of reactions demonstrating microwave-driven de *novo* initiation of RNA synthesis and generation of a double-stranded RNA by RNA polymerases of caliciviruses but not RNA polymerases of other viruses. An ssRNA template of 24 nt was incubated with an RNA polymerase of a sapovirus (lane 1), a norovirus (lane 2), a vesivirus (lane 3), a lagovirus (lane 4), poliovirus (lane 5) or Hepatitis C Virus (lane 6) under microwave irradiation at 800 W for 60 s. Double-stranded products co-migrating with a 25 bp dsRNA marker result with RNA polymerases of caliciviruses (lanes 1 to 4) but not with RNA polymerase of poliovirus (lane 5) or Hepatitis C Virus (lane 6). M: RNA marker corresponding to dsRNA of 17 bp and 25 bp, respectively, and to ssRNA of 24 nt, as indicated.

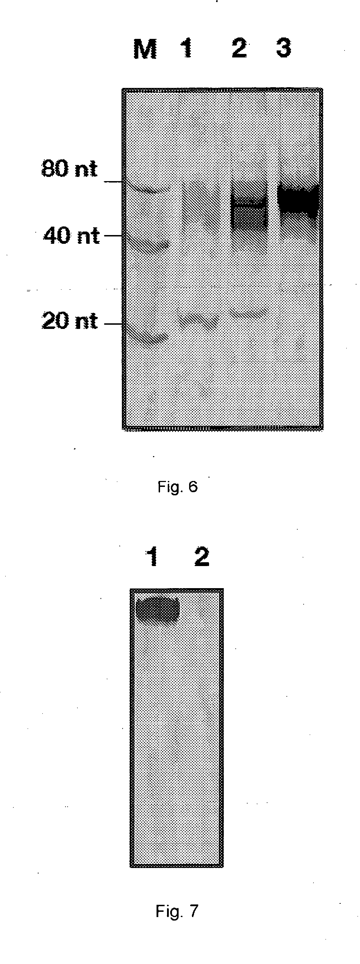

- Fig. 6: shows a photograph of an ethidium bromide-stained native 20% polyacrylamide gel after electrophoretic separation of reactions demonstrating microwave-driven de *novo* initiation of RNA synthesis and generation of a double-stranded DNA/RNA product on ssDNA templates by a sapovirus RNA polymerase. A sapovirus RNA polymerase was incubated with a 24 nt ssDNA template having 5 dC at the 3'-end (lane 1) or a 24 nt ssDNA template of the same sequence but having 5 rC at the 3'-end (lane 3) under microwave irradiation at 800 W for 60 s. As a control, a reaction containing the sapovirus RNA polymerase and an ssRNA template of the same sequence were irradiated with microwaves under the same conditions as before (lane 2). All reactions were carried out in the presence of rATP, rCTP, rUTP and rGTP. A double-stranded product co-migrating with a 25 bp dsRNA marker is visible in lanes 2 and 3, but not in lane 1, indicating that a double-stranded product is generated only in the presence of rC nucleotides at the 3'-end of ssDNA templates. M: RNA marker corresponding to ssDNA of 20 nt, 40 nt and 80 nt, as indicated.
- Fig. 7: shows a photograph of an ethidium bromide-stained native 20% polyacrylamide gel after electrophoretic separation of reactions demonstrating that T7 DNA-dependent RNA polymerase is not capable of RNA synthesis under microwave irradiation. The T7 DNA-dependent RNA polymerase was incubated with a dsDNA template (linearized plasmid including 116 bp of an 18 S rRNA sequence) either at 37°C for 2 h (lane 1) or irradiated with microwaves at 800 W for 60 s (lane 2). All reactions were otherwise carried out under the conditions recommended by the manufacturer of the T7-Megashortscript Kit (Ambion, Inc.). A reaction product is visible after isothermal incubation at 37°C for 2 h (lane 1) but not after microwave irradiation (lane 2).

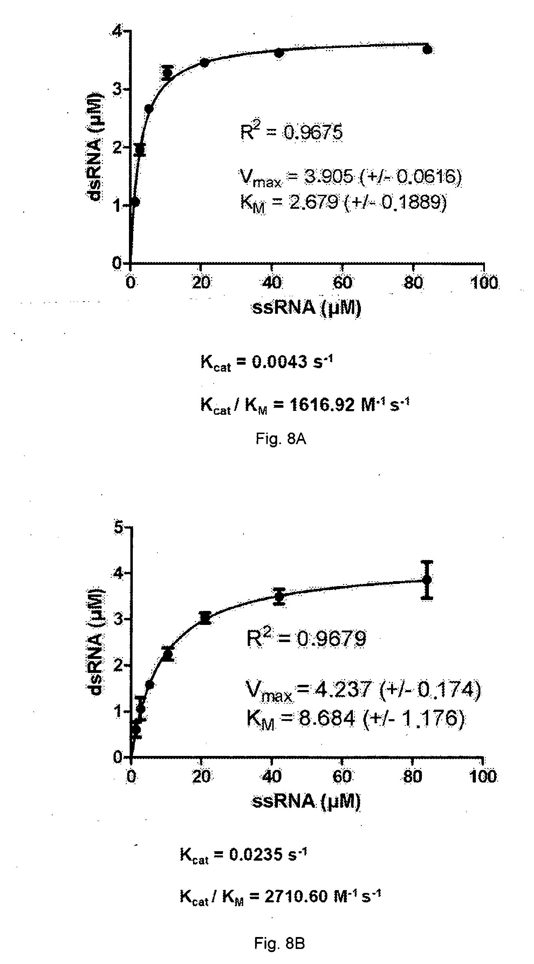

- Fig. 8A: shows a graph reflecting the kinetics of the enzymatic reaction of primer-independent de novo initiation of RNA synthesis by the sapovirus RdRp using heat generated by a thermoblock. For determination of the reaction kinetics, the sapovirus RdRp (SEQ ID NO: 11) was incubated with an RNA template (5'-AUACCUAGAAUCUGACCAACCCCC-3'; SEQ ID NO: 15). The reactions were performed in a total volume of 25 µl disposed in a thermoblock at 37°C. The reaction mix contained 1 µg of the template, 7.5 µM RdRp, 0.4 mM of each ATP, CTP, UTP, and 2 mM GTP, 5 µl reaction buffer (HEPES 250 mM, MnCl₂ 25 mM, DTT 5 mM, pH 7.6), and RNAse-DNAse free water to a total volume of 25 µl.
- Fig. 8B: shows a graph reflecting the kinetics of the enzymatic reaction of primer-independent de novo initiation of RNA synthesis by the sapovirus RdRp using microwave energy. For determination of the reaction kinetics, the sapovirus RdRp (SEQ ID NO: 11) was incubated with an RNA template (5'-AUACCUAGAAUCUGACCAACCCCC-3'; SEQ ID NO: 15). The reactions were performed in a total volume of 25 µl disposed in a microwave at 160 Watt. The reaction mix contained 1 µg of the template, 7.5 µM RdRp, 0.4 mM of each ATP, CTP, UTP, and 2 mM GTP, 5 µl reaction buffer (HEPES 250 mM, MnCl₂ 25 mM, DTT 5 mM, pH 7.6), and RNAse-DNAse free water to a total volume of 25 µl.

The present invention is further illustrated by the following non-limiting examples.

### EXAMPLES

### Example 1: Microwave-driven primer-independent de novo initiation of RNA synthesis and generation of a double-stranded RNA by a sapovirus RNA polymerase

A sapovirus RNA polymerase (SEQ ID NO: 11) was incubated with an RNA template (5'-AUACCUAGAAUCUGACCAACCCCC-3'; SEQ ID NO: 15) under microwave irradiation at 800 W for 60 s in a conventional microwave oven. The sapovirus RNA polymerase generates a double-stranded RNA (see Fig. 1, lane 2) using the single-stranded RNA as a template. The resulting product was incubated with S1 nuclease. No digestion of the product was observed after incubation with S1 nuclease (see Fig. 1, lane 3), indicating the double-stranded nature of the product. All reactions were performed in a total volume of 25 µl. The RNA polymerisation reaction mix contained 1 µg template, 7.5 µM RNA polymerase, 0.4 mM of each ATP, CTP, UTP, and 2 mM GTP, 5 µl reaction buffer (HEPES 250 mM, MnCl₂ 25 mM, DTT 5 mM, pH 7.6), and RNAse-DNAse free water to a total volume of 25 µl. For S1 nuclease digestion, S1 nuclease (250 U) was added to the reaction and the reaction mix incubated for 1 h at 30°C. The products were separated on a native 20% polyacrylamide gel by electrophoresis and visualized by ethidium bromide staining.

### Example 2: Characterisation of microwave power and irradiation time needed for microwave-driven RNA synthesis by calicivirus RNA polymerases

A sapovirus RNA polymerase (SEQ ID NO: 11) was incubated with an RNA template (5'-AUACCUAGAAUCUGACCAACCCCC-3'; SEQ ID NO: 15). The reaction was performed in a total volume of 25 µl disposed in a conventional microwave oven for 60 s at 80 W, 160 W, 240 W, 320 W, 400 W, 480 W, 560 W, 640 W, 720 W and 800 W, respectively. In a further experiment, the reactions were carried out in the same microwave oven at 80 W for 60 s, 30 s, 15 s, or 5 s (see Fig. 2B, lanes 1 to 4), or at 800 W for 60 s, 30 s, 15 s, or 5 s (see Fig. 2B, lanes 5 to 8). A product of the expected size was generated in all reactions (Fig. 2A, 2B).

The reaction mix contained 1 µg template, 7.5 µM RNA polymerase, 0.4 mM of each ATP, CTP, UTP, and 2 mMGTP, 5 µl reaction buffer (HEPES 250 mM, MnCl₂ 25 mM, DTT 5 mM, pH 7.6), and RNAse-DNAse free water to a total volume of 25 µl. The products were separated on a native 20% polyacrylamide gel by electrophoresis and visualized by ethidium bromide staining.

### Example 3: A calicivirus RNA polymerase initiates RNA synthesis on DNA templates de novo in a primer-independent manner and incorporates 2'-fluoro-GMP leading to a double-stranded DNA/RNA product when using microwave irradiation

A sapovirus RNA polymerase (SEQ ID NO: 11) was incubated with an ssDNA template (5'-ATACCTAGAATCTGACCAACCCCC-3'; SEQ ID NO: 16) or a DNA template of the same sequence but having a (rC)₅ sequence motif at the3'-terminus (5'-ATACCTAGAATCTGACCAArCrCrCrCrC-3'; SEQ ID NO: 17). As a control, the sapovirus RNA polymerase was incubated with a single-stranded RNA (5'-AUACCUAGAAUCUGACCAACCCCC-3'; SEQ ID NO: 15) having the same sequence as the above single-stranded DNA. All reactions were performed in a total volume of 25 µl disposed in a conventional microwave oven at 800 W for 60 s. The reaction mix contained 1 µg template, 7.5 µM RNA polymerase, 0.4 mM of each ATP, CTP, UTP, and either GTP (for the control reaction using the ssRNA template; see Fig, 3, lane 3) or 2'-fluoro-GTP (for the reactions using the ssDNA templates; see Fig. 3, lanes 1 and 2), 5 µl reaction buffer (HEPES 250 mM, MnCl₂ 25 mM, DTT 5 mM, pH 7.6), and RNAse-DNAse free water to a total volume of 25 µl. The products were separated on a native 20% polyacrylamide gel by electrophoresis and visualized by ethidium bromide staining.

Fig. 3 (see lanes 1 and 2) demonstrates the microwave-driven RNA synthesis and incorporation of 2'-fluoro-GMP by sapovirus RNA polymerase on ssDNA templates.

### Example 4: A calicivirus RNA polymerase initiates RNA synthesis on DNA templates de novo in a primer-independent manner and incorporates α-thio-GMP leading to a double-stranded DNA/RNA product when using microwave irradiation

A sapovirus RNA polymerase (SEQ ID NO: 11) was incubated with an ssDNA template (5'-ATACCTAGAATCTGACCAACCCCC-3'; SEQ ID NO: 16) or a DNA template of the same sequence but having a (rC)₅ sequence motif at the3'-terminus (5'-ATACCTAGAATCTGACCAArCrCrCrCrC-3'; SEQ ID NO: 17). As a control, the sapovirus RNA polymerase was incubated with a single-stranded RNA (5'-AUACCUAGAAUCUGACCAACCCCC-3'; SEQ ID NO: 15) having the same sequence as the above single-stranded DNA. All reactions were performed in a total volume of 25 µl disposed in a conventional microwave oven at 800 W for 60 s. The reaction mix contained 1 µg template, 7.5 µM RNA polymerase, 0.4 mM of each ATP, CTP, UTP, and either GTP (for the control reaction using the ssRNA template; see Fig. 4, lane 3) or α-thio-GTP (for the reactions using the ssDNA templates; see Fig. 4, lanes 1 and 2), 5 µl reaction buffer (HEPES 250 mM, MnCl2 25 mM, DTT 5 mM, pH 7.6), and RNAse-DNAse free water to a total volume of 25 µl. The products were separated on a native 20% polyacrylamide gel by electrophoresis and visualized by ethidium bromide staining.

Fig. 4 (see lanes 1 and 2) demonstrates the microwave-driven RNA synthesis and incorporation of α-thio-GMP by sapovirus RNA polymerase on ssDNA templates.

### Example 5: Microwave-driven primer-independent de novo initiation of RNA synthesis and generation of a double-stranded RNA by different RNA polymerases of Caliciviridae

A sapovirus RNA polymerase (SEQ ID NO: 11), a norovirus RNA polymerase (SEQ ID NO: 9), a vesivirus RNA polymerase (SEQ ID NO: 13) or a lagovirus RNA polymerase (SEQ ID: 14) was incubated under microwave irradiation with an RNA template (5'-AUACCUAGAAUCUGACCAACCCCC-3'; SEQ ID NO: 15). All calicivirus RNA polymerases generate a double-stranded RNA using single-stranded RNA as a template (Fig. 5, lanes 1 to 4). Furthermore, it was investigated whether RNA polymerases of other viral origin (poliovirus, Hepatitis C Virus) were equally able to perform the same reaction under microwave irradiation. Thus, the poliovirus RNA polymerase and the Hepatitis C Virus RNA polymerase were incubated with the same ssRNA template under microwave irradiation. However, the poliovirus and Hepatitis C Virus RNA polymerases do not generate a double-stranded RNA under the same conditions used for the calicivirus RNA polymerases (see Fig. 5, lanes 5 and 6). All reactions were performed in a total volume of 25 µl disposed in a conventional microwave oven at 800 W for 60 s. The reaction mix contained 1 µg template, 7.5 µM RNA polymerase, 0.4 mM of each ATP, CTP, UTP, and 2 mM GTP, 5 µl reaction buffer (HEPES 250 mM, MnCl₂ 25 mM, DTT 5 mM, pH 7.6), and RNAse-DNAse free water to a total volume of 25 µl. The products were separated on a native 20% polyacrylamide gel by electrophoresis and visualized by ethidium bromide staining.

### Example 6: Microwave-driven primer-independent de novo initiation of RNA synthesis and generation of a DNA-RNA-double strand by the sapovirus RNA polymerase.

The sapovirus RNA polymerase (SEQ ID NO: 11) was incubated with a DNA template (5'-ATACCTAGAATCTGACCAACCCCC-3'; SEQ ID NO: 16) having a (dC)₅ sequence motif at the 3'-terminus, or with a DNA template of the same sequence but havinga (rC)₅ sequence motif at the 3'-terminus (5'-ATACCTAGAATCTGACCAArCrCrCrCrC-3'; SEQ ID NO: 17). As a control, the sapovirus RNA polymerase was incubated with a single-stranded RNA displaying the same sequence as the single-stranded DNA templates (5'-AUACCUAGAAUCUGACCAACCCCC-3'; SEQ ID NO: 15). The sapovirus RNA polymerase generates a DNA/RNA double strand using a single stranded DNA as a template (see Fig. 6, lanes 2 and 3) only in the presence of a C ribonucleotide at the 3'-end of the DNA template. All reactions were performed in a total volume of 25 µl disposed in a conventional microwave oven at 800 W for 60 s. The reaction mix contained 1 µg template, 7.5 µM RNA polymerase, 0.4 mM of each ATP, CTP, UTP, and 2 mM GTP, 5 µl reaction buffer (HEPES 250 mM, MnCl₂ 25 mM, DTT 5 mM, pH 7.6), and RNAse-DNAse free water to a total volume of 25 µl. The products were separated on a native 20% polyacrylamide gel by electrophoresis and visualized by ethidium bromide staining.

### Comparative Example: Comparison of the efficiency of RNA synthesis by the T7 DNA-dependent RNA polymerase (DdRp) under isothermal conditions and under microwave irradiation

The T7 DdRp was used for RNA synthesis on a double-stranded DNA template (linearized plasmid including a 116 bp of a 18S rRNA sequence, 1 µg pro reaction) under recommended isothermal incubation conditions of 37°C for 2 h or using microwave irradiation (800 W for 60 s). The reagents of the T7-Megaschortscritp Kit (Ambion, Inc) were used. All reactions were performed according to the manufacturer's instructions. The product of the reaction incubated under isothermal conditions is shown in Fig, 7, lane 1. In comparison, no product was synthesized when the reaction mixture was exposed to microwave irradiation (800 W for 60 s; Fig. 7, lane 2). Reaction products were separated on a native 20% polyacrylamide gel by electrophoresis and visualized by ethidium bromide staining.

The above Examples and Comparative Example show that RNA polymerases of the virus family of *Caliciviridae* (caliciviruses), but not structurally related RNA-dependent RNA polymerases of other viruses (poliovirus, HCV) or DNA-dependent RNA polymerases (T7 DdRp), polymerise a complementary RNA strand on RNA, DNA or mixed RNA/DNA templates under microwave irradiation.

### Example 7: Increased catalytic efficiency of RNA synthesis by the sapovirus RdRp using microwave energy in comparison to heat generated by a thermoblock

For determination of the reaction kinetics, the sapovirus RdRp (SEQ ID NO: 11) was incubated with an RNA template (5'-AUACCUAGAAUCUGACCAACCCCC-3'; SEQ ID NO: 15). All reactions were performed in a total volume of 25 µl disposed either in a thermoblock at 37°C or in a microwave oven at 160 Watt. The reaction mix contained 1 µg of the template, 7.5 µM RdRp, 0.4 mM of each ATP, CTP, UTP, and 2 mM GTP, 5 µl reaction buffer (HEPES 250 mM, MnCl₂ 25 mM, DTT 5 mM, pH 7.6), and RNAse-DNAse free water to a total volume of 25 µl. Fig. 8A shows the kinetics of the reaction using heat generated by a thermoblock. Fig. 8B shows the kinetics of the reaction using microwave energy. The median +/- SEM of four independent measures are shown (error bars).

The sapovirus RdRp displays an increased catalytic efficiency (K_{cat} up to 5-fold, and K_{cat}/K_{M} up to 1,6-fold) when using microwave as a source of energy in comparison to heat generated by thermoblock.

## Claims

1. A method for polymerising a complementary RNA strand on a single-stranded polynucleotide template comprising irradiating a composition containing said template and an RNA polymerase of a virus of the *Caliciviridae* family under RNA polymerisation conditions in the presence or absence of a primer hybridised to the template, with an effective amount of microwave energy with the proviso that, if said template contains deoxyribonucleotides the irradiation is carried out in the presence of a modified GTP, preferably 2'-fluoro-GTP or α-thio-GTP, and if the template containing deoxyribonucleotides has a deoxyribonucleotide at its 3' end which is not a deoxy-C nucleoteotide, the irradiation step is carried out in the presence of a primer hybridised to the template, and with the further proviso that, if said template is polyadenylated, polyguanylated or polyuridylated RNA or is polyA, polyG or polyU RNA, respectively, the irradiation step is carried out in the presence of a primer hybridised to the template, and if said template is polycytidylated and the irradation step is carried out in the absence of a primer, the composition contains a surplus of GTP with respect to ATP, CTP and UTP, respectively.

2. The method of claim 1 wherein the RNA polymerase is an RNA polymerase of a noroviurs, sapovirus, vesivirus or lagovirus.

3. The method of claim 1 or 2 wherein the RNA polymerase is an RNA polymerase of the norovirus strain HuCV/NUDresden174/1997/GE (GenBank Acc. No AY741811) or an RNA polymerase of the sapovirus strain pJG-Sap01 (GenBank Acc. No AY694184) or an RNA polymerase of the vesivirus strain FCV/Dresden/2006/GE (GenBank Acc. No DQ424892) or an RNA polymerase of the lagovirus strain pJG-RHDV-DD06 (GenBank Acc. No. EF363035.1).

4. The method according to any one of the preceding claims wherein the RNA polymerase has an amino acid sequence selected from the group consisting of SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14.

5. The method according to any one of the preceding claims wherein the composition is irradiated with microwaves having a frequency of from 1500 MHz to 3500 MHz and a power of from 50 to 1000 W.

6. The method according to any one of the preceding claims wherein the composition is irradiated with microwaves for 3 to 120 s.

7. The method according to any one of the preceding claims wherein the composition contains at least one modified or labelled ribonucleotide, optionally besides the modified GTP, preferably 2'-fluoro-GTP or α-thio-GTP.

8. The method according to any one of the preceding claims wherein the template is single-stranded RNA, single-stranded DNA, mixed single-stranded DNA/RNA or a mixture thereof.

9. The method according to any one of the preceding claims wherein the template has a length of from 15 to 30 nucleotides, preferably 21 to 28 nucleotides, more preferably 21 to 23 nucleotides, and the irradiation step is carried out in the absence of a primer.

10. The method according to any one of the preceding claims wherein the template has at least one C nucleotide, preferably 1 to 5 C nucleotides at its 3'-end.

11. The method according to any one of claims 1 to 9 wherein the composition containing the template and the RNA polymerase of a virus of the *Caliciviridae* family is first irradiated with an effective amount of microwave energy in the presence of rCTP as the only nucleotide before carrying out the irradiation step under RNA polymerisation conditions.

12. The method according to any one of the preceding claims wherein the RNA polymerase, under microwave irradiation, separates the double-stranded product into single-strands, synthesises a complementary RNA strand, optionally in the presence of a primer, on each single strand, and repeats the steps of separating complementary strands and RNA synthesis, optionally in the presence of a primer, one or more times.

13. A method for transferring one or more ribonucleotides to the 3' end of a single-stranded polynucleotide template comprising the step of irradiating a composition containing an RNA polymerase of a virus of the *Caliciviridae* family in the presence of rATP or rGTP or rUTP or rCTP or a modified or labelled analogue thereof, with an effective amount of microwave energy.

14. The method of claim 13 wherein the template is single-stranded RNA, single-stranded DNA, mixed single-stranded DNA/RNA or a mixture thereof.

## Patentansprüche

1. Verfahren zum Polymerisieren eines komplementären RNA-Strangs auf einer einzelsträngigen Polynukleotidmatrize, umfassend das Bestrahlen einer Zusammensetzung, welche die Matrize und eine RNA-Polymerase eines Virus aus der Familie der *Caliciviridae* enthält, unter RNA-Polymerisationsbedingungen in Gegenwart oder Abwesenheit eines mit der Matrize hybridisierten Primers, mit einer wirksamen Menge an Mikrowellenenergie mit der Maßgabe, dass, falls die Matrize Desoxyribonukleotide enthält, die Bestrahlung in Gegenwart eines modifizierten GTP, vorzugsweise 2'-Fluor-GTP oder α-Thio-GTP, durchgeführt wird, und, falls die Desoxyribonukleotide enthaltende Matrize ein Desoxyribonukleotid am 3'-Ende aufweist, das kein Desoxy-C-Nukleotid ist, der Bestrahlungsschritt in Gegenwart eines mit der Matrize hybridisierten Primers durchgeführt wird, und mit der weiteren Maßgabe, dass, falls die Matrize polyadenylierte, polyguanylierte oder polyuridylierte RNA bzw. polyA-, polyG-, oder poly-U-RNA ist, der Bestrahlungsschritt in Gegenwart eines mit der Matrize hybridisierten Primers durchgeführt wird, und falls die Matrize polycytidyliert ist und der Bestrahlungsschritt in Abwesenheit eines Primers durchgeführt wird, die Zusammensetzung einen Überschuss an GTP im Vergleich zu ATP, CTP bzw. UTP enthält.

2. Verfahren nach Anspruch 1, wobei die RNA-Polymerase eine RNA-Polymerase eines Norovirus, Sapovirus, Vesivirus oder Lagovirus ist.

3. Verfahren nach Anspruch 1 oder 2, wobei die RNA-Polymerase eine RNA-Polymerase des Norovirus-Stammes HuCV/NL/Dresden174/1997/GE (GenBank Zugriffsnr. AY741811) oder eine RNA-Polymerase des Sapovirus-Stammes pJG-Sap01 (GenBank Zugriffsnr. AY694184) oder eine RNA-Polymerase des Vesivirus-Stammes FCV/Dresden/2006/GE (GenBank Zugriffsnr. DQ424892) oder eine RNA-Polymerase des Lagovirus-Stammes pJG-RHDV-DD06 (GenBank Zugriffsnr. EF363035.1) ist.

4. Verfahren nach einem der vorhergehenden Ansprüche, wobei die RNA-Polymerase eine Aminosäuresequenz aufweist, die aus der Gruppe bestehend aus SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13 und SEQ ID NO: 14 ausgewählt ist.

5. Verfahren nach einem der vorhergehenden Ansprüche, wobei die Zusammensetzung mit Mikrowellen bestrahlt wird, die eine Frequenz von 1500 MHz bis 3500 MHz und eine Leistung von 50 bis 1000 W aufweisen.

6. Verfahren nach einem der vorhergehenden Ansprüche, wobei die Zusammensetzung mit Mikrowellen für 3 bis 120 Sekunden bestrahlt wird.

7. Verfahren nach einem der vorhergehenden Ansprüche, wobei die Zusammensetzung mindestens ein modifiziertes oder markiertes Ribonukleotid, gegebenenfalls neben dem modifizierten GTP vorzugsweise 2'-Fluor-GTP oder α-Thio-GTP, enthält.

8. Verfahren nach einem der vorhergehenden Ansprüche, wobei die Matrize einzelsträngige RNA, einzelsträngige DNA, gemischte einzelsträngige DNA/RNA oder ein Gemisch davon ist.

9. Verfahren nach einem der vorhergehenden Ansprüche, wobei die Matrize eine Länge von 15 bis 30 Nukleotiden, vorzugsweise 21 bis 28 Nukleotiden, mehr bevorzugt 21 bis 23 Nukleotiden, aufweist und der Bestrahlungsschritt in Abwesenheit eines Primers durchgeführt wird.

10. Verfahren nach einem der vorhergehenden Ansprüche, wobei die Matrize mindestens ein C-Nukleotid, vorzugsweise 1 bis 5 C-Nukleotide, am 3'-Ende aufweist.

11. Verfahren nach einem der Ansprüche 1 bis 9, wobei die Zusammensetzung, welche die Matrize und die RNA-Polymerase eines Virus aus der Familie der *Caliciviridae* enthält, zuerst mit einer wirksamen Menge an Mikrowellenenergie in Gegenwart von rCTP als einzigem Nukleotid bestrahlt wird, bevor der Bestrahlungsschritt unter RNA-Polymerisationsbedingungen durchgeführt wird.

12. Verfahren nach einem der vorhergehenden Ansprüche, wobei die RNA-Polymerase unter Mikrowellenbestrahlung das doppelsträngige Produkt in Einzelstränge auftrennt, einen komplementären RNA-Strang, gegebenenfalls in Gegenwart eines Primers, auf jedem Einzelstrang synthetisiert, und die Schritte der Trennung komplementärer Stränge und der RNA-Synthese, gegebenenfalls in Gegenwart eines Primers, ein oder mehrere Male wiederholt.

13. Verfahren zum Anfügen eines oder mehrerer Ribonukleotide an das 3'-Ende einer einzelsträngigen Polynukleotidmatrize, umfassend den Schritt der Bestrahlung einer Zusammensetzung, die eine RNA-Polymerase eines Virus aus der Familie der *Caliciviridae* enthält, in Gegenwart von rATP oder rGTP oder rUTP oder rCTP oder eines modifizierten oder markierten Analogon davon, mit einer wirksamen Menge an Mikrowellenenergie.

14. Verfahren nach Anspruch 13, wobei die Matrize einzelsträngige RNA, einzelsträngige DNA, gemischte einzelsträngige DNA/RNA oder ein Gemisch davon ist.

## Revendications

1. Méthode de polymérisation d'un brin d'ARN complémentaire sur une matrice polynucléotidique simple-brin comprenant l'irradiation d'une composition contenant ladite matrice et une ARN polymérase d'un virus de la famille des *Caliciviridae* dans des conditions de polymérisation de l'ARN, en présence ou en absence d'une amorce hybridée à la matrice, avec une quantité efficace d'énergie micro-onde à condition que, si ladite matrice contient des désoxyribonucléotides, l'irradiation est réalisée en présence d'un GTP modifié, avantageusement du 2'-fluoro-GTP ou de l'α-thio-GTP, et si la matrice contenant les désoxyribonucléotides a un désoxyribonucléotide à son extrémité 3' qui n'est pas un désoxy-C nucléotide, l'étape d'irradiation est réalisée en présence d'une amorce s'hybridant à la matrice, et à la condition supplémentaire que, si ladite matrice est polyadénylée, polyguanylée ou de l'ARN polyuridylé ou est de l'ARN polyA, polyG ou polyU respectivement, l'étape d'irradiation est réalisée en présence d'une amorce s'hybridant à la matrice, et si la matrice est polycytidylée et l'étape d'irradiation est réalisée en l'absence d'une amorce, la composition contient un excédent de GTP par rapport à l'ATP, le CTP et l'UTP, respectivement.

2. Méthode selon la revendication 1, dans laquelle l'ARN polymérase est une ARN polymérase d'un norovirus, sapovirus, vesivirus ou lagovirus.

3. Méthode selon la revendication 1 ou 2, dans laquelle l'ARN polymérase est une ARN polymérase de la souche de norovirus HuCV/NL/Dresden174/1997/GE (GenBank Acc. No AY741811) ou une ARN polymérase de la souche de sapovirus pJG-Sap01 (GenBank Acc. No AY694184) ou une ARN polymérase de la souche de vesivirus FCV/Dresden/2006/GE (GenBank Acc. No DQ424892) ou une ARN polymérase de la souche de lagovirus pJG-RHDV-DD06 (GenBank Acc. No EF363035.1).

4. Méthode selon l'une des revendications précédentes, dans laquelle l'ARN polymérase a une séquence en acides aminés sélectionnée dans le groupe constitué de SEQ ID NO : 6, SEQ ID NO :7, SEQ ID NO :8, SEQ ID NO :9, SEQ ID NO :10, SEQ ID NO :11, SEQ ID NO :12, SEQ ID NO :13 et SEQ ID NO :14.

5. Méthode selon l'une des revendications précédentes, dans laquelle la composition est irradiée avec des micro-ondes ayant une fréquence allant de 1500 MHz à 3500 MHz et une puissance allant de 50 à 1000 W.

6. Méthode selon l'une des revendications précédentes, dans laquelle la composition est irradiée avec des micro-ondes pendant 3 à 120 s.

7. Méthode selon l'une des revendications précédentes, dans laquelle la composition contient au moins un ribonucléotide modifié ou marqué, optionnellement en plus du GTP modifié, avantageusement du 2'-fluoro-GTP ou de l'α-thio-GTP.

8. Méthode selon l'une des revendications précédentes, dans laquelle la matrice est un ARN simple-brin, un ADN simple-brin, un mixte ADN/ARN simple-brin ou un mélange de ceux-ci.

9. Méthode selon l'une des revendications précédentes, dans laquelle la matrice a une longueur de 15 à 30 nucléotides, avantageusement 21 à 28 nucléotides, encore plus avantageusement 21 à 23 nucléotides, et l'étape d'irradiation est réalisée en l'absence d'une amorce.

10. Méthode selon l'une des revendications précédentes, dans laquelle la matrice comporte au moins un nucléotide C, avantageusement 1 à 5 nucléotides C, à son extrémité 3'.

11. Méthode selon l'une des revendications 1 à 9, dans laquelle la composition contenant la matrice et l'ARN polymérase d'un virus de la famille des *Caliciviridae* est d'abord irradiée avec une quantité efficace d'énergie micro-onde en présence de rCTP en tant que seul nucléotide, avant de réaliser l'étape d'irradiation dans des conditions de polymérisation de l'ARN.

12. Méthode selon l'une des revendications précédentes, dans laquelle l'ARN polymérase, sous irradiation micro-onde, sépare les produits double-brins en simple brins, synthétise un brin d'ARN complémentaire, optionnellement en présence d'une amorce à partir de chaque brin simple, et répète les étapes de séparation des brins complémentaires et la synthèse d'ARN, optionnellement en présence d'une amorce, une à plusieurs fois.

13. Méthode pour transférer un ou plusieurs ribonucléotides à l'extrémité 3' d'une matrice polynucléotidique simple-brin comprenant l'étape d'irradiation d'une composition comprenant une ARN polymérase d'un virus de la famille des *Caliciviridae,* en présence de rATP ou rGTP ou rUTP ou rCTP ou d'un analogue modifié ou marqué de ceux-ci, avec une quantité efficace d'énergie micro-onde.

14. Méthode selon la revendication 13, dans laquelle la matrice est un ARN simple-brin, un ADN simple-brin, un ADN/ARN simple-brin mixte ou un mélange de ceux-ci.
